# EUROPÄISCHE PATENTANMELDUNG

(11) **EP 1 642 561 A1**
(43) Veröffentlichungstag der Anmeldung: **05.04.2006**
(21) Anmeldenummer: 04023278.7
(22) Anmeldetag: 30.09.2004
(51) Int. Cl.: A61K 8/18, A61K 8/99, A61K 47/30

(54) **Kosmetische Mittel enthaltend sulfonierte, Sulfongruppen enthaltende Polymere**

(71) Anmelder: BASF Aktiengesellschaft, 67056 Ludwigshafen (DE)
(72) Erfinder: Angel, Maximilian Dr., 67105 Schifferstadt (DE); Möhwald, Helmut Dr., 76855 Annweiler (DE); Wood, Claudia Dr., 69469 Weinheim (DE); Mathauer, Klemens Dr., 69115 Heidelberg (DE)

(57) **Zusammenfassung**

Die vorliegende Erfindung betrifft kosmetische und pharmazeutische Mittel enthaltend sulfonierte Polysulfone, Polyethersulfone und Polyphenylsulfone und die Verwendung der sulfonierten Polysulfone, Polyethersulfone und Polyphenylsulfone in kosmetischen und pharmazeutischen Zubereitungen.

## Beschreibung

Die vorliegende Erfindung betrifft kosmetische Mittel enthaltend sulfonierte, Sulfongruppen enthaltende Polymere und in der Kosmetik übliche Zusatzstoffe.

### Stand der Technik

Sulfonierte, Sulfongruppen enthaltende Polymere werden beispielsweise in der Brennstoffzellentechnik als Bestandteile von Polymerelektrolytmembranen verwendet. Diese sulfonierten Polysulfone sind hierbei derart funktionalisiert, dass sie zum lonenaustausch und dabei bevorzugt zur Aufnahme und Abgabe von Protonen befähigt sind.

Weiterhin werden sulfonierte, Sulfongruppen enthaltende Polymere im Bereich der Mundhygiene verwendet. EP-A 045 194 beschreibt die Verwendung von sulfonierten Polyarylethersulfonen als Barrierematerialien gegen Plaquebildung.

Die Verwendung von sulfonierten, Sulfongruppen enthaltende Polymeren in kosmetischen oder dermatologischen Mitteln ist nicht bekannt.

### Aufgabe der Erfindung

In der Kosmetik, insbesondere bei kosmetischen Zubereitungen, besteht ein Bedarf an Polymeren, die in für kosmetische Zubereitungen üblichen Lösungsmitteln wie beispielsweise Wasser, Alkoholen oder Gemischen daraus löslich sind, die gute Eigenschaften als Filmbildner zeigen und als Filme geringe Klebrigkeit und gute mechanische Eigenschaften aufweisen.

In kosmetischen Zubereitungen für die Haut können Polymere besondere Wirkung entfalten, z.B. zur Feuchthaltung und Konditionierung der Haut und zur Verbesserung des Hautgefühls. Die erfindungsgemäßen kosmetischen Zubereitungen sind insbesondere für die Anwendung auf der Epidermis geeignet.

In kosmetischen Zubereitungen für das Haar werden Polymere zur Festigung, Strukturverbesserung und Formgebung der Haare verwendet. Sie erhöhen die Kämmbarkeit und verbessern den Griff des Haares.

Nachteil zahlreicher Polymere, die als Filmbildner geeignet sind, ist deren ungenügende Löslichkeit in in der Kosmetik üblichen Lösungsmitteln. Um dieses Problem zu beheben, werden teilweise aufwendige Maßnahmen wie Copolymerisation hydrophober und hydrophiler Monomere zur Einstellung der Löslichkeit vorgenommen. Die resultierenden Filme sind häufig klebrig und weisen nicht die gewünschten mechanischen Eigenschaften auf.

Aufgabe der vorliegenden Erfindung war es daher, Polymere für die Verwendung in kosmetischen Zubereitungen bereitzustellen, die in für kosmetische Zubereitungen üblichen Lösungsmitteln löslich sind und klare Filme mit möglichst geringer Klebrigkeit und guten mechanischen Eigenschaften bilden können.

Diese Aufgabe wurde gelöst durch die Bereitstellung von kosmetischen Mitteln enthaltend sulfonierte, Sulfongruppen enthaltende Polymere und in der Kosmetik übliche Zusatzstoffe.

### Sulfongruppen enthaltende Polymere

Unter Sulfongruppen enthaltenden Polymeren werden in diesem Zusammenhang Polymere verstanden, die eine para-gebundene Diphenylensulfon-Struktureinheit der allgemeinen Formel I als Teil der Polymerkette enthalten, wobei die Phenylenreste dieser Struktureinheit gegebenenfalls ein- oder mehrfach substituiert sind.

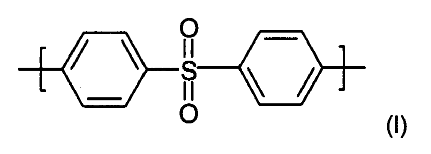

Besonders geeignet für die Verwendung in kosmetischen, insbesonders haar- und hautkosmetischen sowie pharmazeutischen Zubereitungen sind die sulfonierten, Sulfongruppen enthaltenden Polymeren.

Weiterer Gegenstand der Erfindung sind haar- und hautkosmetische Mittel enthaltend sulfonierte Polysulfone sowie die Verwendung von sulfonierten, Sulfongruppen enthaltenden Polymeren in kosmetischen und pharmazeutischen Zubereitungen.

Darüberhinaus können die sulfonierten, Sulfongruppen enthaltenden Polymere in Mitteln für die Mund- und Zahnpflege verwendet werden. Ein weiterer Gegenstand der Erfindung sind also Mittel zur Mund- und Zahnpflege enthaltend sulfonierte, Sulfongruppen enthaltende Polymere und weitere für Zubereitungen der Mund- und Zahnpflege übliche Zusätze.

Als Edukte für die Herstellung der sulfonierten, Sulfongruppen enthaltenden Polymere geeignete Polymere sind beispielsweise in der EP-A 045 194, Seite 6, Zeile 19 bis Seite 10, Zeile 2 beschrieben, worauf an dieser Stelle vollumfänglich Bezug genommen wird.

Besonders geeignet für die Verwendung in kosmetischen Mitteln sind sulfonierte, Sulfongruppen enthaltende Polymere ausgewählt aus sulfonierten Polysulfonen (abgekürzt als PSU), sulfonierten Polyethersulfonen (abgekürzt als PES) und sulfonierten Polyphenylsulfonen (abgekürzt als PPSU).

Diese sulfonierten, Sulfongruppen enthaltenden Polymere werden bevorzugt aus geeigneten Sulfongruppen enthaltenden Polymeren durch Sulfonierung hergestellt. Solche geeigneten Sulfongruppen enthaltende Polymere sind beispielsweise:
PSU wie beispielsweise Udel® (Hersteller: Solvay), Ultrason®S (Hersteller: BASF) und Gafone®S (Hersteller: Gharda Chemicals);
PES wie beispielsweise Ultrason®E (Hersteller: BASF), Gafone® (Hersteller: Gharda Chemicals), Radel®A (Hersteller: Solvay), Vitrex® (Hersteller: ICI) und Sumikaexcel® (Hersteller: Sumitomo);
PPSU wie beispielsweise Radel®R (Hersteller: Solvay), Victrex®HTA (Hersteller: ICI) oder Gafone®P (Hersteller: Gharda Chemicals).

Herstellung der sulfonierten, Sulfongruppen enthaltenden Polymere
Die Sulfonierung der Sulfongruppen enthaltenden Polymere erfolgt auf übliche, dem Fachmann bekannte Art und Weise.

Beispielsweise offenbart die EP-A 045 194 auf Seite 11, Zeile 31 bis Seite 15, Zeile 11 die Sulfonierung und anschließende Aufarbeitung der Sulfongruppen enthaltenden Polymere und in den Beispielen 1, 2 und 5 speziell die Sulfonierung von Udel®, wobei unterschiedliche Sulfonierungsgrade erreicht werden. Beispiel 6 beschreibt die Herstellung eines PPSU-Natriumsalzes und Beispiel 7 die Herstellung eines PSU mit hohem Sulfonierungsgrad (130%) auf Basis von Radel®. Die in den Beispielen der EP-A 045 194 genannten sulfonierten, Sulfongruppen enthaltenden Polymere sind für die Verwendung in den erfindungsgemäßen Mitteln geeignet.

Prinzipiell sind sämtliche aus dem Stand der Technik bekannten Sulfonierungsmittel wie Oleum, konzentrierte Schwefelsäure, hochkonzentrierte (d.h. ca. 98%ige) Schwefelsäure, Schwefeltrioxid oder Chlorsulfonsäure in mindestens einem geeigneten organischen Lösungsmittel oder Butyllithium zusammen mit Schwefeldioxid mit nachfolgender Oxidation mittels beispielsweise Kaliumpermanganat einsetzbar.

Sulfonierte, Sulfongruppen enthaltende Polymere können weiterhin auch durch Polykondensation von sulfonierten Diarylsulfonderivaten mit Biphenolen hergestellt werden. Journal of Membrane Science 197 (2002) 231-242 beschreibt die Umsetzung von 3,3'-Disulfonat-4,4'-Dichlor-diphenylsulfon (SDCDPS) mit 4,4'-Dichlor-diphenylsulfon (DCDPS) und 4,4'-Biphenol.

### Sulfonierungsgrad

Ein Sulfonierungsgrad von 100 % bezeichnet hierbei ein Sulfongruppen enthaltendes Polymer, das im statistischen Mittel pro Wiederholungseinheit des Polymers eine Sulfonatgruppe aufweist (der Begriff "Sulfonatgruppe" bezeichnet sowohl die protonierte (-SO₃H) als auch jede neutralisierte Form davon). Sulfonierungsgrade von über 100% kennzeichnen Sulfongruppen enthaltende Polymere, die mehr als eine Sulfonatgruppe pro Wiederholungseinheit des Polymers enthalten.

Dabei steht der Ausdruck Sulfonatgruppe für eine Struktureinheit -SO₃Mₓ, wobei für x=1 M Wasserstoff oder einwertige Kationen wie beispielsweise die Kationen von Li, Na, K, NH₄ bedeutet, für x=1/2 M zweiwertige Kationen wie beispielsweise die Kationen von Mg, Ca, Zn bedeutet und für x=1/3 M dreiwertige Kationen wie beispielsweise das Kation von Al bedeutet.

Im folgenden werden unter der Wortwahl "sulfonierte, Sulfongruppen enthaltende Polymere" sowohl die neutrale, also die protonierte Form, als auch jegliche Salzform der sulfonierten, Sulfongruppen enthaltenden Polymere verstanden.

Sulfonierte, Sulfongruppen enthaltende Polymere mit hohem Sulfonierungsgrad, z.B. 80 % und größer sind wasserlöslich und solche mit sehr niedrigem Sulfonierungsgrad, z.B. unter 40 % sind schlecht wasserlöslich. Vorteilhaft für die Verwendung der sulfonierten, Sulfongruppen enthaltenden Polymere in kosmetischen Mitteln sind wasserlösliche bzw. in wässrigen Lösungsmittelgemischen lösliche Polymere.

Die erfindungsgemäßen kosmetischen Mittel enthalten bevorzugt sulfonierte, Sulfongruppen enthaltende Polymere mit einem Sulfonierungsgrad über 50%, insbesondere über 70%, besonders bevorzugt über 80%.

### Neutralisierung der sulfonierten, Sulfongruppen enthaltenden Polymere

Eine bevorzugte Ausführungsform der Erfindung sind kosmetische Mittel, die sulfonierte, Sulfongruppen enthaltende Polymere enthalten, welche in teilweise oder vollständig neutralisierter Form vorliegen.

Bei bevorzugten teilweise neutralisierten Polymeren ist die Menge der SO₃H-Einheiten beispielsweise mindestens zu 30, bevorzugt mindestens zu 40, besonders bevorzugt mindestens zu 50, ganz besonders bevorzugt mindestens zu 70 und insbesondere mindestens zu 95 % neutralisiert.

In einer weiteren bevorzugten Ausführungsform ist die Menge der SO₃H-Einheiten zu mindestens 99% neutralisiert.

Diese teilweise oder vollständig neutralisierte Form kann in üblicher Weise durch Umsetzung der Polymere mit organischen oder anorganischen Basen erhalten werden.

Als Neutralisierungsmittel sind erfindungsgemäß alle Basen geeignet, mit denen die sulfonierten, Sulfongruppen enthaltenden Polymere kosmetisch, dermatologisch und/oder pharmazeutisch akzeptable Salze bilden.

Die Neutralisierung erfolgt vorteilhaft mit
- einem Mono-, Di- oder Trialkanolamin mit 2 bis 5 Kohlenstoffatomen im Alkanolrest, der gegebenenfalls in veretherter Form vorliegt, beispielsweise Mono-, Di- und Triethanolamin, Mono-, Di- und Tri-n-propanolamin, Mono-, Di- und Triisopropanolamin, 2-Amino-2-methylpropanol und Di(2-methoxyethyl)amin,
- einem Alkandiolamin mit 2 bis 5 Kohlenstoffatomen, beispielsweise 2-Amino-2-methylpropan-1,3-diol und 2-Amino-2-ethylpropan-1,3-diol, oder
- einem primären, sekundären oder tertiären Alkylamin mit insgesamt 5 bis 10 Kohlenstoffatomen, beispielsweise N,N-Diethylpropylamin oder 3-Diethylamino-1-propylamin.

Häufig werden gute Neutralisationsergebnisse mit 2-Amino-2-methylpropanol, Triisopropanolamin, 2-Amino-2-ethylpropan-1,3-diol oder 3-Diethylamino-1-propylamin erhalten. In einer besonders bevorzugten Ausführungsform der Erfindung wird Triethanolamin zur Neutralisation verwendet.

Die sulfonierten, Sulfongruppen enthaltenden Polymere können auch mit Metallhydroxiden, Ammoniak oder Ammoniumhydroxid neutralisiert werden. Bevorzugt sind kosmetische Mittel enthaltend sulfonierte, Sulfongruppen enthaltende Polymere, welche teilweise oder vollständig als Ammonium- und/oder Alkalimetallsalze vorliegen.

In einer bevorzugten Ausführungsform werden die sulfonierten, Sulfongruppen enthaltenden Polymere mit MOH neutralisiert, wobei M ausgewählt ist aus Alkalimetallen oder Ammonium (NH₄⁺-, Monoalkylammonium-, Dialkylammonium-, Trialkylammonium- und Tetraalkylammoniumionen), bevorzugt ausgewählt aus Li, Na, K, NH₄, besonders bevorzugt aus Na, K, NH₄.

In einer weiteren bevorzugten Ausführungsform werden die sulfonierten, Sulfongruppen enthaltenden Polymere mit entsprechenden Hydroxiden von kosmetisch, dermatologisch und/oder pharmazeutisch akzeptablen zwei- oder dreiwertigen Metallen neutralisiert. Bevorzugt sind die kosmetisch, dermatologisch und/oder pharmazeutisch akzeptablen Hydroxide der Metalle der 2. und 3. Hauptgruppe des Periodensystems der Elemente. Insbesondere seien die Hydroxide von Mg, Ca und Al genannt.

Weiterhin eignen sich zur Neutralisation wässrige Pufferlösungen, wie beispielsweise Puffer basierend auf Alkali- bzw. Ammoniumcarbonat oder -bicarbonat.

Die Neutralisationsmittel werden vorzugsweise als verdünnte wässrige Lösung eingesetzt. Bevorzugt ist die Verwendung von sulfonierten, Sulfongruppen enthaltenden Polymere, die teilweise oder vollständig neutralisiert werden.

Neutralisationsgrad und Neutralisationsmittel beeinflussen maßgeblich die Löslichkeit und die mechanischen Eigenschaften der aus den Lösungen der sulfonierten, Sulfongruppen enthaltenden Polymere herstellbaren Filme.

Beispielsweise besitzen Filme, die aus mit Calciumhydroxid neutralisierten sulfonierten, Sulfongruppen enthaltenden Polymeren hergestellt werden, andere mechanische Eigenschaften, beispielsweise einen größeren E-Modul als Filme, die aus mit Triethanolamin neutralisierten sulfonierten, Sulfongruppen enthaltenden Polymeren hergestellt werden.

Erfindungsgemäß können demnach die Löslichkeit der sulfonierten, Sulfongruppen enthaltenden Polymeren und die mechanischen Eigenschaften der aus den Lösungen herstellbaren Filme durch den Neutralisationsgrad und die Wahl des zur Neutralisation eingesetzten Neutralisationsmittels eingestellt werden.

Die mechanischen Eigenschaften hängen demnach auch von der Art der Gegenionen ab. Besonders vorteilhaft ist es deshalb, die mechanischen Eigenschaften durch die Wahl der Gegenionen zu beeinflussen.

Es ist auch möglich, eine Salzform der sulfonierten, Sulfongruppen enthaltenden Polymere in eine andere Salzform zu überführen, also beispielsweise ein Natriumsulfonatgruppen tragendes, Sulfongruppen enthaltendes Polymer in ein Zinksulfonatgruppen tragendes Polymer zu überführen. Eine derartige Überführung ist beispielsweise in EP-A 045 194, Beispiel 3 beschrieben.

### Kosmetische Zubereitungen

In den erfindungsgemäßen kosmetischen Zubereitungen liegen die sulfonierten, Sulfongruppen enthaltenden Polymere vorteilhaft in gelöster Form vor. Als Lösungsmittel kommen Wasser, ein- und mehrwertige Alkohole und Mischungen daraus in Betracht.

Bevorzugte Lösungsmittel sind Wasser, Ethanol, n-Propanol, Isopropanol, n-Butanol, Butan-2-ol, 2-Methylpropan-1-ol, 2-Methylpropan-2-ol, Propylenglykol, Glycerin, Sorbit sowie Mischungen daraus.

In einer besonders bevorzugten Ausführungsform ist das Lösungsmittel ausgewählt aus der Gruppe bestehend aus Wasser, Ethanol, Isopropanol und Mischungen daraus.

Die erfindungsgemäßen kosmetischen Mittel enthaltend sulfonierte, Sulfongruppen enthaltende Polymere können als wässrige oder wässrig-alkoholische Lösungen, O/Wsowie W/O-Emulsionen in Form von Shampoos, Cremes, Schäumen, Sprays (Pumpspray oder Aerosol), Gelen, Gelsprays, Lotionen oder Mousse vorliegen und dementsprechend mit üblichen weiteren Hilfsstoffen formuliert werden.

### Zusätze

Die kosmetischen, insbesondere die haar- und hautkosmetischen Zubereitungen können neben den sulfonierten, Sulfongruppen enthaltenden Polymeren und geeigneten Lösungsmitteln noch in derartigen Formulierungen übliche Zusätze Emulgatoren und Co-Emulgatoren, Tenside, Ölkörper, Konservierungsmittel, Parfümöle, kosmetische Pflege- und Wirkstoffe wie AHA-Säuren, Fruchtsäuren, Ceramide, Phytantriol, Collagen, Vitamine und Provitamine, beispielsweise Vitamin A, E und C, Retinol, Bisabolol, Panthenol, natürliche und synthetische Lichtschutzmittel, Naturstoffe, Trübungsmittel, Lösungsvermittler, Repellents, Bleichmittel, Färbemittel, Tönungsmittel, Bräunungsmittel (z.B. Dihydroxyaceton), Mikropigmente wie Titanoxid oder Zinkoxid, Überfettungsmittel, Perlglanzwachse, Konsistenzgeber, Verdicker, Solubilisatoren, Komplexbildner, Fette, Wachse, Silikonverbindungen, Hydrotrope, Farbstoffe, Stabilisatoren, pH-Wert Regulatoren, Reflektoren, Proteine und Proteinhydrolysate (z.B. Weizen-, Mandel- oder Erbsenproteine), Ceramid, Eiweißhydrolysate, Salze, Gelbildner, Konsistenzgeber, Silikone, Feuchthaltemittel, Rückfetter und weitere übliche Additive enthalten. Des weiteren können zur Einstellung der jeweils gewünschten Eigenschaften insbesondere auch weitere Polymere enthalten sein.

Zum Schutz der Haut und der Haare vor Beeinträchtigungen durch UV-Strahlung können in den kosmetischen Zubereitungen auch UV-Lichtschutzmittel enthalten sein.

Beispiele für die jeweiligen Klassen von Hilfsstoffen sind im folgenden genannt, ohne dabei die möglichen Hilfsstoffe auf die beispielhaft genannten zu begrenzen.

Übliche Zusätze für kosmetische Zubereitungen sind dem Fachmann beispielsweise aus W. Umbach, "Kosmetik", 2. Auflage, Thieme-Verlag 1995, ISBN 3-13-712602-9, bekannt.

### Reinigung der Haut

Erfindungsgemäß werden die sulfonierten, Sulfongruppen enthaltenden Polymere beispielsweise in kosmetischen Mitteln zur Reinigung der Haut verwendet. Solche kosmetischen Reinigungsmittel sind ausgewählt aus Stückseifen, wie Toilettenseifen, Kernseifen, Transparentseifen, Luxusseifen, Deoseifen, Cremeseifen, Babyseifen, Hautschutzseifen, Abrasivseifen und Syndets, flüssigen Seifen, wie pastöse Seifen, Schmierseifen und Waschpasten, und flüssigen Wasch-, Dusch- und Badepräparaten, wie Waschlotionen, Duschbädern und -gelen, Schaumbädern, Ölbädern und Scrub-Präparaten.

### Pflege und Schutz der Haut

Bevorzugt werden die sulfonierten, Sulfongruppen enthaltenden Polymere in kosmetischen Mitteln zur Pflege und zum Schutz der Haut, in Nagelpflegemitteln sowie in Zubereitungen für die dekorative Kosmetik angewendet.

Besonders bevorzugt ist die Verwendung in Hautpflegemitteln, Intimpflegemitteln, Fußpflegemitteln, Deodorantien, Lichtschutzmitteln, Repellents, Rasiermitteln, Haarentfernungsmitteln, Antiaknemitteln, Make-up, Maskara, Lippenstifte, Lidschatten, Kajalstiften, Eyelinern, Rouges, Pudern und Augenbrauenstiften.

Die Hautpflegemittel liegen insbesondere als W/O- oder O/W-Hautcremes, Tag- und Nachtcremes, Augencremes, Gesichtscremes, Antifaltencremes, Feuchthaltecremes, Bleichcremes, Vitamincremes, Hautlotionen, Pflegelotionen und Feuchthaltelotionen vor.

In den kosmetischen Zubereitungen können die sulfonierten, Sulfongruppen enthaltenden Polymere besondere Wirkungen entfalten. Die sulfonierten, Sulfongruppen enthaltenden Polymere können unter anderem zur Feuchthaltung und Konditionierung der Haut und zur Verbesserung des Hautgefühls beitragen. Die sulfonierten, Sulfongruppen enthaltenden Polymere können auch als Verdicker in den Formulierungen wirken. Durch Zusatz der sulfonierten, Sulfongruppen enthaltenden Polymere kann in bestimmten Formulierungen eine erhebliche Verbesserung der Hautverträglichkeit erreicht werden.

Die sulfonierten, Sulfongruppen enthaltenden Polymere sind in den hautkosmetischen Zubereitungen in einem Anteil von etwa 0,001 bis 20 Gew.-%, vorzugsweise 0,01 bis 10 Gew.-%, ganz besonders bevorzugt 0,1 bis 5 Gew.-%, bezogen auf das Gesamtgewicht des Mittels, enthalten.

Gegenstand der Erfindung sind demnach auch hautkosmetische Mittel enthaltend sulfonierte, Sulfongruppen enthaltende Polymere.

Je nach Anwendungsgebiet können die erfindungsgemäßen Mittel in einer zur Hautpflege geeigneten Form, wie z.B. als Creme, Schaum, Gel, Stift, Pulver, Mousse, Milch oder Lotion appliziert werden.

Die hautkosmetischen Zubereitungen können neben den sulfonierten, Sulfongruppen enthaltenden Polymere und geeigneten Lösungsmitteln noch weitere in der Kosmetik übliche Zusätze, wie Emulgatoren, Konservierungsmittel, Parfümöle, kosmetische Wirkstoffe wie Phytantriol, Vitamin A, E und C, Retinol, Bisabolol, Panthenol, Lichtschutzmittel, Bleichmittel, Färbemittel, Tönungsmittel, Bräunungsmittel (z.B. Dihydroxyaceton), Collagen, Eiweißhydrolysate, Stabilisatoren, pH-Wert-Regulatoren, Farbstoffe, Salze, Verdicker, Gelbildner, Konsistenzgeber, Silikone, Feuchthaltemittel, Rückfetter und weitere übliche Additive enthalten.

Als weitere übliche Zusätze können enthalten sein Fettkörper, wie mineralische und synthetische Öle, wie z.B. Paraffine, Siliconöle und aliphatische Kohlenwasserstoffe mit mehr als 8 Kohlenstoffatomen, tierische und pflanzliche Öle, wie z.B. Sonnenblumenöl, Kokosöl, Avocadoöl, Olivenöl, Lanolin, oder Wachse, Fettsäuren, Fettsäureester, wie z.B. Triglyceride von C₆-C₃₀-Fettsäuren, Wachsester, wie z.B. Jojobaöl, Fettälkohole, Vaseline, hydriertes Lanolin und acetyliertes Lanolin. Selbstverständlich können auch Mischungen derselben verwendet werden.

Übliche Verdickungsmittel in derartigen Formulierungen sind vernetzte Polyacrylsäuren und deren Derivate, Polysacharide wie Xanthan-Gum, Agar-Agar, Alginate oder Tylosen, Carboxymethylcellulose oder Hydroxycarboxymethylcellulose, Fettalkohole, Monoglyceride und Fettsäuren, Polyvinylakolhol und Polyvinylpyrrolidon.

Selbstverständlich können die kosmetischen, insbesondere auch die hautkosmetischen Zubereitungen neben den sulfonierten, Sulfongruppen enthaltenden Polymeren weitere herkömmliche Polymere enthalten, um bestimmte Eigenschaften und Wirkungen der Zubereitungen zu erreichen.

Als herkömmliche Polymere eignen sich beispielsweise anionische, kationische, amphotere und neutrale Polymere.

Beispiele für anionische Polymere sind Homo- und Copolymerisate von Acrylsäure und Methacrylsäure oder deren Salze, Copolymere von Acrylsäure und Acrylamid und deren Salze; Natriumsalze von Polyhydroxycarbonsäuren, wasserlösliche oder wasserdispergierbare Polyester, Polyurethane und Polyharnstoffe. Besonders geeignete Polymere sind Copolymere aus t-Butylacrylat, Ethylacrylat, Methacrylsäure (z.B. Luvimer™ 100P), Copolymere aus Ethylacrylat und Methacrylsäure (z.B. Luvimer™ MAE), Copolymere aus N-tert.-Butyl-acrylamid, Ethylacrylat, Acrylsäure (Ultrahold™ 8, strong), Copolymere aus Vinylacetat, Crotonsäure und gegebenenfalls weitere Vinylester (z.B. Luviset™ Marken), Maleinsäureanhydridcopolymere, ggf. mit Alkoholen umgesetzt, anionische Polysiloxane, z.B. carboxyfunktionelle, Copolymere aus Vinylpyrrolidon, t-Butylacrylat, Methacrylsäure (z.B Luviskol™ VBM), Copolymere von Acrylsäure und Methacrylsäure mit hydrophoben Monomeren, wie z.B. C₄-C₃₀-Alkylester der Meth(acrylsäure), C₄-C₃₀-Alkylvinylester, C₄-C₃₀-Alkylvinylether und Hyaluronsäure, Luviset® P.U.R., Luviflex® Silk.

Weitere geeignete Polymere sind kationische Polymere mit der Bezeichnung Polyquaternium nach INCI, z.B. Copolymere aus Vinylpyrrolidon/N-Vinylimidazoliumsalzen (Luviquat™ FC, Luviquat™ HM, Luviquat™ MS, Luviquat™ Care, Luviquat™ Hold, INCI Polyquaternium-16, -44, -46), Copolymere aus Acrylamid und Dimethyldiallylammoniumchlorid (Polyquaternium-7), kationische Cellulosederivate (Polyquaternium-4, -10), kationische Stärkederivate (INCI: Starch Hydroxypropytrimonium Chloride, Corn Starch Modified), kationische Guarderivate (INCI: Hydroxypropyl Guar Hydroxypropyltrimonium Chloride), kationische Sonnenblumenöl-Derivate (INCI: Sunflowerseedamidopropyl Hydroxyethyldimonium Chloride), Copolymere aus N-Vinypyrrolidon/ Dimethylaminoethylmethacrylat, quaternisiert mit Diethylsulfat (Polyquaternium-11), Copolymere aus Acrylsäure, Acrylamid und Methacrylamidopropyltrimoniumchlorid (Polyquaternium-53), Polyquaternium-32, Polyquaternium-28 und andere.

Als weitere Polymere sind auch neutrale Polymere geeignet wie Polyvinylpyrrolidone, Copolymere aus N-Vinylpyrrolidon und Vinylacetat und/oder Vinylpropionat, Copolymere aus N-Vinypyrrolidon/Dimethylaminopropylacrylamid oder -methacrylamid, Copolymere aus N-Vinylpyrrolidon und Alkylacrylat- oder -methacrylatmonomeren mit Alkylketten von C₁ bis C₁₈, Pfropfcopolymere von Polyvinylalkohol auf Polyalkylenglykole wie z.B. Kollicoat□ IR (BASF), Pfropfcopolymere von anderen Vinylmonomeren auf Polyalkylenglykole, Polysiloxane, Polyvinylcaprolactam und Copolymere mit N-Vinylpyrrolidon, Polyethylenimine und deren Salze, Polyvinylamine und deren Salze, Cellulosederivate, Chitosan, Polyasparaginsäuresalze und Derivate.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze, Dimethicone, Dimethicone-Derivate oder Dimethicon Copolyole (CTFA) und aminofunktionelle Silikonverbindungen wie Amodimethicone (CTFA).

Die sulfonierten, Sulfongruppen enthaltenden Polymere werden in kosmetischen Zubereitungen eingesetzt, deren Herstellung nach den üblichen dem Fachmann geläufigen Regeln erfolgt.

Die sulfonierten, Sulfongruppen enthaltenden Polymere werden zur Herstellung der kosmetischen Zubereitungen bevorzugt in gelöster Form bereitgestellt. Insbesondere werden die sulfonierten, Sulfongruppen enthaltenden Polymere in wässriger Lösung oder in wässrig/alkoholischer Lösung, besonders bevorzugt in wässriger Lösung bereitgestellt.

Solche Formulierungen liegen vorteilhafterweise in Form von Emulsionen bevorzugt als Wasser-in-Öl- (W/O)- oder Öl-in-Wasser-(O/W)-Emulsionen vor. Es ist aber auch erfindungsgemäß möglich und gegebenenfalls vorteilhaft, andere Formulierungsarten zu wählen, beispielsweise Hydrodispersionen, Gele, Öle, Oleogele, multiple Emulsionen, beispielsweise in Form von W/O/W- oder O/W/O-Emulsionen, wasserfreie Salben bzw. Salbengrundlagen usw.

Die Herstellung erfindungsgemäß brauchbarer Emulsionen erfolgt nach bekannten Methoden.

Die Emulsionen enthalten neben den sulfonierten, Sulfongruppen enthaltenden Polymeren übliche Bestandteile, wie Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser.

Die Auswahl der Emulsionstyp-spezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, Dritter Teil, worauf hiermit ausdrücklich Bezug genommen wird.

So kann eine erfindungsgemäß brauchbare Hautcreme z.B. als W/O-Emulsion vorliegen. Eine derartige Emulsion enthält eine wässrige Phase, die mittels eines geeigneten Emulgatorsystems in einer Öl- oder Fettphase emulgiert ist.

Die Konzentration des Emulgatorsystems beträgt in diesem Emulsions-Typ etwa 4 und 35 Gew.-%, bezogen auf das Gesamtgewicht der Emulsion; die Fettphase macht etwa 20 und 60 Gew.-% aus und die wässrige Phasen etwa 20 und 70 Gew.-%, jeweils bezogen auf das Gesamtgewicht der Emulsion. Bei den Emulgatoren handelt es sich um diejenigen, welche in diesem Emulsionstyp üblicherweise verwendet werden. Sie werden z.B. ausgewählt unter: C₁₂-C₁₈-Sorbitan-Fettsäureestern; Estern von Hydroxystearinsäure und C₁₂-C₃₀-Fettalkoholen; Mono- und Diestern von C₁₂-C₁₈-Fettsäuren und Glyzerin oder Polyglyzerin; Kondensaten von Ethylenoxid und Propylenglycolen; oxypropylenierten/oxyethylenierten C₁₂-C₂₀-Fettalkoholen; polycyclischen Alkoholen, wie Sterolen; aliphatischen Alkoholen mit einem hohen Molekulargewicht, wie Lanolin; Mischungen von oxypropylenierten/polyglycerinierten Alkoholen und Magnesiumisostearat; Succinestern von polyoxyethylenierten oder polyoxypropylenierten Fettalkoholen; und Mischungen von Magnesium-, Calcium-, Lithium-, Zink- oder Aluminiumlanolat und hydriertem Lanolin oder Lanolin-alkohol.

Zu geeigneten Fettkomponenten, welche in der Fettphase der Emulsionen enthalten sein können, zählen Kohlenwasserstofföle, wie Paraffinöl, Purcellinöl, Perhydrosqualen und Lösungen mikrokristalliner Wachse in diesen Ölen; tierische oder pflanzliche Öle, wie Süßmandelöl, Avocadoöl, Calophylumöl, Lanolin und Derivate davon, Ricinusöl, Sesamöl, Olivenöl, Jojobaöl, Karité-Öl, Hoplostethus-Öl; mineralische Öle, deren Destillationsbeginn unter Atmosphärendruck bei ca. 250°C und deren Destillationsendpunkt bei 410°C liegt, wie z.B. Vaselinöl; Ester gesättigter oder ungesättigter Fettsäuren, wie Alkylmyristate, z.B. i-Propyl-, Butyl- oder Cetylmyristat, Hexadecylstearat, Ethyl- oder i-Propylpalmitat, Octan- oder Decansäuretriglyceride und Cetylricinoleat. Die Fettphase kann auch in anderen Ölen lösliche Siliconöle, wie Dimethylpolysiloxan, Methylphenylpolysiloxan und das Silikonglycol-Copolymer, Fettsäuren und Fettalkohole enthalten.

Um die Retention von Ölen zu begünstigen, kann man auch Wachse verwenden, wie z.B. Carnauba-Wachs, Candellilawachs, Bienenwachs, mikrokristallines Wachs, Ozokeritwachs und Ca-, Mg- und Al-Oleate, -Myristate, -Linoleate und -Stearate.

Im allgemeinen werden diese Wasser-in-ÖI-Emulsionen so hergestellt, dass die Fettphase und der Emulgator in den Ansatzbehälter gegeben werden. Man erwärmt diesen bei einer Temperatur von 70 bis 75°C, gibt dann die in Öl löslichen Ingredienzen zu und fügt unter Rühren Wasser hinzu, welches vorher auf die gleiche Temperatur erwärmt wurde und worin man die wasserlöslichen Ingredienzen vorher gelöst hat; man rührt, bis man eine Emulsion der gewünschten Feinheit hat, lässt sie dann auf Raumtemperatur abkühlen, wobei gegebenenfalls weniger gerührt wird.

Weiterhin kann eine erfindungsgemäße Pflegeemulsion als O/W-Emulsion vorliegen. Eine derartige Emulsion enthält üblicherweise eine Ölphase, Emulgatoren, die die Ölphase in der Wasserphase stabilisieren, und eine wässrige Phase, die üblicherweise verdickt vorliegt.

Die wässrige Phase der O/W-Emulsion der erfindungsgemäßen Zubereitungen enthält gegebenenfalls
- Alkohole, Diole oder Polyole sowie deren Ether, vorzugsweise Ethanol, Isopropanol, Propylenglycol, Glycerin, Ethylenglycolmonoethylether;
- übliche Verdickungsmittel bzw. Gelbildner, wie z.B. vernetzte Polyacrylsäuren und deren Derivate, Polysaccharide wie Xanthan Gum oder Alginate, Carboxymethylcellulose oder Hydroxycarboxymethylcellulose, Fettalkohole, Polyvinylalkohol und Polyvinylpyrrolidon.

Die Ölphase enthält in der Kosmetik übliche Ölkomponenten, wie beispielsweise:
- Ester aus gesättigten und/oder ungesättigten, verzweigten und/oder unverzweigten C₃-C₃₀-Alkancarbonsäuren und gesättigten und/oder ungesättigten, verzweigten und/oder unverzweigten C₃-C₃₀-Alkoholen, aus aromatischen Carbonsäuren und gesättigten und/oder ungesättigten, verzweigten und/oder unverzweigten C₃₋C₃₀-Alkoholen, beispielhaft Isopropylmyristat, Isopropylstearat, Hexyldecylstearat, Oleyloleat; außerdem synthetische, halbsynthetische und natürliche Gemische solcher Ester, wie Jojobaöl;
- verzweigte und/oder unverzweigte Kohlenwasserstoffe und -wachse;
- Silikonöle wie Cyclomethicon, Dimethylpolysiloxan, Diethylpolysiloxan, Octamethylcyclotetrasiloxan sowie Mischungen daraus;
- Dialkylether;
- Mineralöle und Mineralwachse;
- Triglyceride gesättigter und/oder ungesättigter, verzweigter und/oder unverzweigter C₈-C₂₄-Alkancarbonsäuren; sie können ausgewählt werden aus synthetischen, halbsynthetischen oder natürlichen Ölen, wie Olivenöl, Palmöl, Mandelöl oder Mischungen.

Als Emulgatoren kommen vorzugsweise O/W-Emulgatoren, wie Polyglycerinester, Sorbitanester oder teilveresterte Glyceride, in Betracht.

Die Herstellung kann durch Aufschmelzen der Ölphase bei ca. 80°C erfolgen; die wasserlöslichen Bestandteile werden in heißem Wasser gelöst, langsam und unter Rühren zur Ölphase zugegeben; homogenisiert und kaltgerührt.

Die sulfonierten, Sulfongruppen enthaltenden Polymere eignen sich erfindungsgemäß auch zur Verwendung in Wasch- und Duschgel-Formulierungen sowie Badepräparaten.

Solche Formulierungen enthalten neben den sulfonierten, Sulfongruppen enthaltenden Polymeren üblicherweise anionische Tenside als Basistenside und amphotere und nichtionische Tenside als Cotenside, sowie Lipide, Parfümöle, Farbstoffe, organische Säuren, Konservierungsstoffe und Antioxidantien sowie Verdicker/Gelbildner, Hautkonditioniermittel und Feuchthaltemittel.

In den Wasch, Dusch- und Badepräparaten können alle in Körperreinigungsmitteln üblicherweise eingesetzte anionische, neutrale, amphotere oder kationische Tenside verwendet werden.

Die Formulierungen enthalten 2 bis 50 Gew.-% Tenside, bevorzugt 5 bis 40 Gew.-%, besonders bevorzugt 8 bis 30 Gew-%.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Alkylglykolalkoxylate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxideinheiten im Molekül aufweisen.

Geeignet sind zum Beispiel Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate- oder -propionate, Alkylamphodiacetate, oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäure-ester von Polyethylenglykolen, ethoxylierte Fettsäureamide, Alkylpolyglykoside oder Sorbitanetherester geeignet.

Außerdem können die Wasch, Dusch- und Badepräparate übliche kationische Tenside enthalten, wie z.B. quaternäre Ammoniumverbindungen, beispielsweise Cetyltrimethylammoniumchlorid oder -bromid (INCI Cetrimoniumchloride oder -bromide), Hydroxyethylcetyldimoniumphosphat (INCI Quaternium-44), INCI Cocotrimoniummethosulfate, INCI Quaternium-52.

Zusätzlich können auch weitere übliche kationische Polymere eingesetzt werden, so z.B. Copolymere aus Acrylamid und Dimethyldiallylammoniumchlorid (Polyquaternium-7), kationische Cellulosederivate (Polyquaternium-4, -10), kationische Stärkederivate (INCI: Starch Hydroxypropytrimonium Chloride, Corn Starch Modified), kationische Guarderivate (INCI: Hydroxypropyl Guar Hydroxypropyltrimonium Chloride), kationische Sonnenblumenöl-Derivate (INCI: Sunflowerseedamidopropyl Hydroxyethyldimonium Chloride), Copolymere aus N-Vinylpyrrolidon und quaternisiertem N-Vinylimidazol (Polyquaternium-16, -44, -46), Copolymere aus N-Vinypyrrolidon/Dimethylaminoethylmethacrylat, quaternisiert mit Diethylsulfat (Polyquaternium-11), Copolymere aus Acrylsäure, Acrylamid und Methacrylamidopropyltrimoniumchlorid (Polyquaternium-53), Polyquaternium-32, Polyquaternium-28 und andere.

Weiterhin können Wasch- und Duschgel-Formulierungen und Badepräparate Verdicker, wie z.B. Kochsalz, PEG-55, Propylene Glycol Oleate, PEG-120 Methyl Glucose Dioleate und andere, sowie Konservierungsmittel, weitere Wirk- und Hilfsstoffe und Wasser enthalten.

### Haarkosmetische Zubereitungen

Haarkosmetische Zubereitungen umfassen insbesondere Stylingmittel und/oder Konditioniermittel in haarkosmetischen Zubereitungen wie Haarkuren, Haarschäume (engl. Mousses), (Haar)gele oder Haarsprays, Haarlotionen, Haarspülungen, Haarshampoos, Haaremulsionen, Spitzenfluids, Egalisierungsmittel für Dauerwellen, Haarfärbe- und - bleichmittel, "Hot-Oil-Treatment"-Präparate, Conditioner, Festigerlotionen oder Haarsprays. Je nach Anwendungsgebiet können die haarkosmetischen Zubereitungen als (Aerosol-) Spray, (Aerosol-)Schaum, Gel, Gelspray, Creme, Lotion, Milch oder Wachs appliziert werden.

Ein weiterer Gegenstand der Erfindung sind somit kosmetische Mittel, wobei das Mittel als Gel, Milch, Creme, Lotion oder Schaum vorliegt. Insbesondere liegt das Mittel als Gel oder Schaum vor.

Bevorzugt handelt es sich bei dem Mittel um ein Produkt für das Haar, das unter den Haarwaschmitteln und Produkten für das Haar ausgewählt ist, die ausgespült oder nicht ausgespült werden und vor oder nach einer Haarwäsche, Färbung, Entfärbung, Dauerwelle oder Entkräuselung aufgetragen werden.

Erfindungsgemäß ist auch ein Verfahren zur Behandlung von Haaren, dadurch gekennzeichnet, daß die Haare mit dem kosmetisches Mittel, enthaltend ein sulfoniertes, Sulfongruppen enthaltendes Polymer, in Kontakt gebracht werden und gegebenenfalls mit Wasser gespült wird.

Die erfindungsgemäßen haarkosmetischen Zubereitungen enthalten in einer bevorzugten Ausführungsform
a) 0,05 bis 20 Gew.-% sulfoniertes, Sulfongruppen enthaltendes Polymer
b) 20 bis 99,95 Gew.-% Wasser und/oder Alkohol
c) 0 bis 79,5 Gew.-% weitere Bestandteile

Unter Alkohol sind alle in der Kosmetik üblichen Alkohole zu verstehen, z.B. Ethanol, Isopropanol, n-Propanol.

Unter weiteren Bestandteilen sind die in der Kosmetik üblichen Zusätze zu verstehen, beispielsweise Treibmittel, Entschäumer, grenzflächenaktive Verbindungen, d.h. Tenside, Emulgatoren, Schaumbildner und Solubilisatoren. Die eingesetzten grenzflächenaktiven Verbindungen können anionisch, kationisch, amphoter oder neutral sein. Weitere übliche Bestandteile können ferner sein z.B. Konservierungsmittel, Parfümöle, Weichmacher, Effektstoffe, Trübungsmittel, Wirkstoffe, Antioxidantien, Peroxidzersetzer, UV-Filter, Pflegestoffe wie Panthenol, Collagen, Vitamine, Eiweißhydrolysate, Alpha- und Beta-Hydroxycarbonsäuren, Eiweißhydrolysate, Stabilisatoren, pH-Wert-Regulatoren, Farbstoffe, Pigmente, Viskositätsregulierer, Gelbildner, Salze, Feuchthaltemittel, Rückfetter, Komplexbildner und weitere übliche Additive.

Bei Bedarf können selbstverständlich die für hautkosmetische Zubereitungen geeigneten Zusatzstoffe auch in den haarkosmetischen Zubereitungen verwendet werden.

### Styling- und Conditionerpolymere

Weiterhin zählen hierzu alle in der Kosmetik bekannten Styling- und Conditionerpolymere, die in Kombination mit den erfindungsgemäßen Polymerisaten eingesetzt werden können, falls ganz spezielle Eigenschaften eingestellt werden sollen. Als herkömmliche Polymere eignen sich dazu beispielsweise anionische, kationische, amphotere und neutrale Polymere.

Bevorzugte Beispiele für solche weiteren Polymere sind
- Copolymere aus tert.-Butylacrylat, Ethylacrylat und Methacrylsäure
- Copolymere aus Ethylacrylat und Methacrylsäure
- Copolymere aus N-tert.-Butylacrylamid, Ethylacrylat und Acrylsäure
- Polyvinylpyrrolidone
- Polyvinylcaprolactame
- Polyurethane, wie beispielsweise in WO 94/03510 beschrieben
- Polyharnstoffe
- Polyamide
- Copolymere aus Acrylsäure, Methylmethacrylat, Octylacrylamid, Butylaminoethylmethylacrylat und Hydroxypropylmethacrylat,
- Copolymere aus Vinylacetat und Crotonsäure und/oder (Vinyl)-Neodecanoat,
- Copolymere aus Vinylacetat und/oder Vinylpropionat und N-Vinylpyrrolidon,
- carboxyfunktionelle Copolymere aus Vinylpyrrolidon, t-Butylacrylat, Methacrylsäure,
- Copolymere aus tert.-Butylacrylat, Methacrylsäure und Dimethicone Copolyol.
   Überraschenderweise wurde gefunden, dass Zubereitungen, welche die sulfonierten, Sulfongruppen enthaltenden Polymere in Kombination mit diesen weiteren Polymeren enthalten, unerwartete Eigenschaften aufweisen. Die erfindungsgemäßen Zubereitungen sind insbesondere hinsichtlich ihrer haut- und haarpflegenden Eigenschaften den Zubereitungen des Standes der Technik überlegen. Weiterhin weisen sie sehr gute filmbildende und festigende Eigenschaften auf.
   Copolymere aus tert.-Butylacrylat, Ethylacrylat und Methacrylsäure (INCI Bezeichnung: Acrylates Copolymer) sind beispielsweise als Handelsprodukt Luvimer® 100 P, Luvimer® 36 D, Luvimer® 30 E (BASF) erhältlich.
   Copolymere aus Ethylacrylat und Methacrylsäure (INCI Bezeichnung: Acrylates Copolymer), sind beispielsweise als Handelsprodukte Luviflex® Soft (BASF) erhältlich.
   Copolymere aus N-tert.-Butylacrylamid, Ethylacrylat und Acrylsäure (INCI Bezeichnung: Acrylates/Acrylamide Copolymer) sind beispielsweise als Handelsprodukte Ultrahold Strong®, Ultrahold 8® (BASF) erhältlich.
   Polyvinylpyrrolidone (INCI Bezeichnung: PVP) sind beispielsweise unter den Handelsnamen Luviskol® K, Luviskol® K 30 (BASF) Luviskol® K17 , Luviskol® K30, Luviskol® K60, Luviskol® K80, Luviskol® K90 (Polyvinylpyrrolidone mit entsprechendem K-Wert als Pulver oder als Lösung (wässrig oder wässrig/alkoholisch) und PVP K (ISP) erhältlich.
   Polyvinylcaprolactame (INCI: Polyvinylcaprolactame) sind beispielsweise unter dem Handelsnamen Luviskol Plus® (BASF) erhältlich.
   Polyurethane (INCI: Polyurethane -1) sind beispielsweise unter dem Handelsnamen Luviset® PUR erhältlich.
   Copolymere aus Acrylsäure, Methylmethacrylat, Octylacrylamid, Butylaminoethylmethylacrylat, Hydroxypropylmethacrylat (INCI: Octylacrylamide/Acrylates/Butylaminoethyl Methacrylate Copolymer) sind beispielsweise unter den Handelsnamen Amphomer® 28-4910 und Amphomer® LV-71 (National Starch) bekannt.
   Copolymere aus Vinylacetat und Crotonsäure (INCI: VA/Crotonate/Copolymer) sind beispielsweise unter den Handelsnamen Luviset CA 66° (BASF), Resyn® 28-1310 (National Starch) und Aristoflex® A (Celanese) erhältlich.

### Copolymere aus Vinylacetat, Crotonsäure und (Vinyl)neodecanoate (INCI:

VA/Crotonates/Neodecanoate Copolymer) sind beispielsweise unter den Handelsnamen Resyn□ 28-2930 (National Starch) und Luviset® CAN (BASF) erhältlich. Copolymere aus Vinylacetat und N-Vinylpyrrolidon (INCI: PVPNA) sind beispielsweise unter den Handelsnamen Luviskol VA® (BASF), insbesondere Luviskol® VA 73 , Luviskol® VA 64, Luviskol® VA 55, Luviskol® VA 37, Luviskol® VA 28 und PVPNA (ISP) erhältlich.

Carboxyfunktionelle Copolymere aus Vinylpyrrolidon, t-Butylacrylat, Methacrylsäure sind beipielsweise unter dem Handelsnamen Luviskol™ VBM (BASF) erhältlich.

Weiterhin als zusätzliche Polymere geeignet sind Terpolymere aus Vinylpyrrolidon, Vinylacetat und Vinylpropionat, die beispielsweise unter der Bezeichnung Luviskol® VAP 343 erhältlich sind.

Copolymere aus tert.-Butylacrylat, Methacrylsäure und Dimethicone Copolyol sind beipielsweise unter dem Handelsnamen Luviflex™ Silk (BASF) erhältlich. Diese Polymere sowie ihre Herstellung sind in der WO 99/04750 beschrieben.

Weitere zur Verwendung in den erfindungsgemäßen kosmetischen Zubereitungen geeignete Polymere sind Homo- und Copolymere von monoolefinisch ungesättigten Monomeren wie vor allem Homo- und Copolymere von C₁-C₁₂-Alkylacrylaten und -methacrylaten, monoolefinisch ungesättigten C₃-C₅-Monocarbonsäuren und deren Vinylestern, Maleinsäure und deren Halbestern, Methylvinylether, Acrylamiden, Methacrylamiden, N-Alkylsubstituierten(meth)acrylamiden, N-Vinylpyrrolidon, N-Vinylimidazol sowie Styrol.

Als weitere Polymere zur Verwendung in den erfindungsgemäßen kosmetischen Zubereitungen eignen sich beispielsweise anionische Polymere. Solche anionischen Polymere sind von den erfindungsgemäßen (Meth)Acrylatpolymerisaten verschiedene Homo- und Copolymerisate von Acrylsäure und Methacrylsäure oder deren Salze, Copolymere von Acrylsäure und Acrylamid und deren Salze, Natriumsalze von Polystyrolsulfonsäure, Natriumsalze von Polyhydroxycarbonsäuren, Copolymere von Acrylsäure und Methacrylsäure mit beispielsweise hydrophoben Monomeren, z.B. C₄-C₃₀₋Alkylester der (Meth)acrylsäure, C₄-C₃₀-Alkylvinylester, C₄-C₃₀-Alkylvinylether und Hyaluronsäure so wie weitere unter den Handelsnamen Amerhold® DR-25, Ultrahold®, Luviset® P.U.R., Acronal®, Acudyne®, Lovocryl®, Versatyl®, Amphomer® (28-4910, LV-71), Placise® L53, Flexan® 130, Gantrez® ES 425, Advantage Plus®, Omnirez® 2000, Resyn® 28-1310, Resyn® 28-2930, Balance® (0/55), Acydyne® 255, Aristoflex® A oder Eastman AQ® Balance 47 (National Starch; Octylacrylamid/Acrylat/Butylaminoethylmethacrylate-Copolymer), Aquaflex® FX 64 (ISP; Isobutylen/Ethylmaleimid/Hydroxyethylmaleimid-Copolymer), Aquaflex® SF-40 (ISP / National Starch; VP/Vinyl Caprolactam/DMAPA Acrylatcopolymer), Allianz®LT-120 (ISP / Rohm & Haas; Acrylat/C1-2 Succinat/Hydroxyacrylat-Copolymer), Aquarez□ HS (Eastman; Polyester-1), Diaformer® Z-400 (Clariant; Methacryloylethylbetain/Methacrylat-Copolymer), Diaformer® Z-711 oder Z-712 (Clariant; Methacryloylethyl N-oxid/Methacrylat-Copolymer), Omnirez® 2000 (ISP; Monoethylester von Poly(Methylvinylether/Maleinsäure in Ethanol), Amphomer® HC oder Resyn® XP oder Resyn® 28-4961 (National Starch; Acrylat/Octylacrylamide Copolymer), Amphomer® 28-4910 (National Starch; Octylacrylamid/Acrylat/Butylaminoethylmethacrylat-Copolymer), Advantage^{□} HC 37 (ISP; Terpolymer aus Vinylcaprolactam/Vinylpyrrolidon/ Dimethylaminoethylmethacrylat), Advantage® Marken (ISP), Acudyne® 258 (Rohm & Haas; Acrylat/ Hydroxyesteracrylat-Copolymer), Luviset® P.U.R. (BASF, Polyurethane-1), Luviflex® Silk (BASF, PEG/PPG-25/25 Dimethicone/Acrylates Copolymer), Eastman® AQ48 (Eastman), Styleze® 2000 (ISP; VP/Acrylates/Lauryl Methacrylate Copolymer), Styleze® CC-10 (ISP; VP/DMAPA Acrylates Copolymer), Styleze® W-20 (ISP), Fixomer® A-30 (Ondeo Nalco; Methacrylic Acid/Sodium Acrylamidomethyl Propane Sulfonate Copolymer), Fixate® G-100 (Noveon; AMP-Acrylates/Allyl Methacrylate Copolymer).

Als zusätzliche Polymere sind weiterhin geeignet wasserlösliche oder wasserdispergierbare Polyester, Polyharnstoffe, Co-Polyurethanharnstoffe, gegebenenfalls mit Alkoholen umgesetzte Maleinsäureanhydridcopolymere oder anionische, z.B. carboxyfunktionelle Polysiloxane.

Als zusätzliche weiterhin geeignete Polymere sind z.B. auch kationische Polymere mit der INCI-Bezeichnung Polyquaternium wie beispielsweise
- Copolymere aus N-Vinylpyrrolidon/N-Vinylimidazoliumsalzen (erhältlich beispielsweise unter den Handelsnamen Luviquat® FC, Luviquat® HM, Luviquat®MS, Luviquat® Care (BASF)),
- Copolymere aus N-Vinylcaprolactam/N-Vinylpyrrolidon/N-Vinylimidazoliumsalzen (erhältlich beispielsweise unter dem Handelsnamen Luviquat®Hold),
- Copolymere aus N-Vinylpyrrolidon/Dimethylaminoethylmethacrylat, quaternisiert mit Diethylsulfat (erhältlich beispielsweise unter dem Handelsnamen Luviquat® PQ11),
- kationische Cellulosederivate (Polyquatemium-4 und -10),
- Acrylamidcopolymere (Polyquaternium-7),
- Styleeze® CC-10, Aquaflex® SF-40,
- Polyethylenimine und deren Salze,
- Polyvinylamine und deren Salze Copolymere aus N-Vinylpyrrolidon/Dimethylaminoethylmethacrylat, quaternisiert mit Diethylsulfat (Luviquat® PQ 11, INCI: Polyquaternium-11),
- Copolymere aus Acrylamid und Dimethyldiallylammoniumchlorid (Polyquaternium-7),
- kationische Cellulosederivate (Polyquaternium-4, -10),
- kationische Stärkederivate (INCI: Starch Hydroxypropytrimonium Chloride, Corn Starch Modified),
- kationische Guarderivate (INCI: Hydroxypropyl Guar Hydroxypropyltrimonium Chloride),
- kationische Sonnenblumenöl-Derivate (INCI: Sunflowerseedamidopropyl Hydroxyethyldimonium Chloride),
- Copolymere aus Acrylsäure, Acrylamid und Methacrylamidopropyltrimoniumchlorid (INCI: Polyquaternium-53),
- Polyquaternium-32,
- Polyquaternium-28 und andere.

Als weitere Haarkosmetik-Polymere sind auch neutrale Polymere geeignet wie Polyvinylpyrrolidon, Copolymere aus N-Vinylpyrrolidon und Vinylacetat und/oder Vinylpropionat, Copolymere aus N-Vinypyrrolidon/Dimethylaminopropylacrylamid oder -methacrylamid, Copolymere aus N-Vinylpyrrolidon und Alkylacrylat- oder -methacrylatmonomeren mit Alkylketten von C₁ bis C₁₈ Polysiloxane, Pfropfcopolymere von Polyvinylalkohol auf Polyalkylenglykole wie z.B. Kollicoat® IR, Pfropfcopolymere von anderen Vinylmonomeren auf Polyalkylenglykole, Polysiloxane, Polyvinylcaprolactam und Copolymere mit N-Vinylpyrrolidon, Polyethylenimine und deren Salze, Polyvinylamine und deren Salze, Polyasparaginsäuresalze und Derivate.

Dazu gehören die unter den Handelsnamen bekannten Luviskol® (K, VA, Plus), PVP K, PVP/VA, Advantage®HC und H₂OLD® EP-1.

Außerdem geeignet sind auch Biopolymere, d.h. Polymere, die aus natürlich nachwachsenden Rohstoffen gewonnen werden und aus natürlichen Monomerbausteinen aufgebaut sind, z.B. Cellulosederivate, Chitin-, Chitosan-, DNA-, Hyaluronsäure- und RNA-Derivate.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze, Dimethicone, Dimethicone-Derivate oder Dimethicon Copolyole (CTFA) und aminofunktionelle Silikonverbindungen wie Amodimethicone (CTFA).

Weitere geeignete Polymere sind auch betaine Polymere wie Yukaformer® (R205, SM) und Diaformer®.

Die erfindungsgemäßen Polymerisate eignen sich insbesondere als Festigungsmittel in Haarstyling-Zubereitungen, insbesondere Haarsprays (Aerosolsprays und Pumpsprays ohne Treibgas) und Haarschäume (Aerosolschäume und Pumpschäume ohne Treibgas).

Ein weiterer Gegenstand der Erfindung ist somit die Konfektionierung des kosmetischen Mittels in einem Zerstäuber, Pumpflakon oder Aerosolbehälter um ein Spray, einen Lack oder einen Schaum zu erhalten.

Bevorzugt ist dabei die Konfektionierung des kosmetischen Mittels in einem Pumpzerstäuber.

In einer bevorzugten Ausführungsform enthalten diese Zubereitungen
a) 0,1 bis 10 Gew.-% der sulfonierten, Sulfongruppen enthaltenden Polymere
b) 20 bis 99,9 Gew.-% Wasser und/oder Alkohol
c) 0 bis 70 Gew.-% eines Treibmittel
d) 0 bis 20 Gew.-% weitere Bestandteile

Treibmittel sind die für Haarsprays oder Aerosolschäume üblich verwendeten Treibmittel. Bevorzugt sind Gemische aus Propan/Butan, Pentan, Dimethylether, 1,1-Difluorethan (HFC-152 a), Kohlendioxid, Stickstoff oder Druckluft.

Eine erfindungsgemäß bevorzugte Formulierung für Aerosolhaarschäume enthält
a) 0,1 bis 10 Gew.-% sulfonierte, Sulfongruppen enthaltende Polymere
b) 55 bis 99,8 Gew.-% Wasser und/oder Alkohol
c) 5 bis 20 Gew.-% eines Treibmittel
d) 0,1 bis 5 Gew.-% eines Emulgators
e) 0 bis 10 Gew.-% weitere Bestandteile

Als Emulgatoren können alle in Haarschäumen üblicherweise eingesetzten Emulgatoren verwendet werden. Geeignete Emulgatoren können nichtionisch, kationisch bzw. anionisch oder amphoter sein.

Beispiele für nichtionische Emulgatoren (INCI-Nomenklatur) sind Laurethe, z.B. Laureth-4; Cetethe, z.B. Cetheth-1, Polyethylenglycolcetylether; Cetearethe, z.B. Cetheareth-25, Polyglycolfettsäureglyceride, hydroxyliertes Lecithin, Lactylester von Fettsäuren, Alkylpolyglycoside.

Beispiele für kationische Emulgatoren sind Cetyltrimethylammoniumchlorid oder - bromid (INCI Cetrimoniumchloride oder -bromide), Hydroxyethylcetyldimoniumphosphat (INCI Quaternium-44), INCI Cocotrimoniummethosulfate, INCI Quaternium-52, Quaternium-1 bis x (INCI).

Anionische Emulgatoren können beispielsweise ausgewählt werden aus der Gruppe der Alkylsulfate, Alkylethersulfate, Alkylsulfonate, Alkylarylsulfonate, Alkylsuccinate, Alkylsulfosuccinate, N-Alkoylsarkosinate, Alkylglykolalkoxylate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Eine erfindungsgemäß für Styling-Gele geeignete Zubereitung kann beispielsweise wie folgt zusammengesetzt sein:
a) 0,1 bis 10 Gew.-% der sulfonierte, Sulfongruppen enthaltende Polymere
b) 60 bis 99,85 Gew.-% Wasser und/oder Alkohol
c) 0,05 bis 10 Gew.-% eines Gelbildners
d) 0 bis 20 Gew.-% weitere Bestandteile

Als Gelbildner können alle in der Kosmetik üblichen Gelbildner eingesetzt werden. Hierzu zählen leicht vernetzte Polyacrylsäure, beispielsweise Carbomer (INCI), Cellulosederivate, z.B. Hydroxypropylcellulose, Hydroxyethylcellulose, kationisch modifizierte Cellulosen, Polysaccharide, z.B. Xanthum Gummi, Caprylic/Capric Triglyceride, Sodium acrylates Copolymer, Polyquaternium-32 (and) Paraffinum Liquidum (INCI), Sodium Acrylates Copolymer (and) Paraffinum Liquidum (and) PPG-1 Trideceth-6, Acrylamidopropyl Trimonium Chloride/Acrylamide Copolymer, Steareth-10 Allyl Ether Acrylates Copolymer, Polyquaternium-37 (and) Paraffinum Liquidum (and) PPG-1 Trideceth-6, Polyquaternium 37 (and) Propylene Glycole Dicaprate Dicaprylate (and) PPG-1 Trideceth-6, Polyquaternium-7, Polyquaternium-44.

Die sulfonierten, Sulfongruppen enthaltenden Polymere können in kosmetischen Zubereitungen als Konditioniermittel und /oder als Verdicker eingesetzt werden.

Die sulfonierten, Sulfongruppen enthaltenden Polymere können erfindungsgemäß auch in Shampooformulierungen als Festigungs- und/oder Konditioniermittel eingesetzt werden.

Bevorzugte Shampooformulierungen enthalten
a) 0,05 bis 10 Gew.-% der sulfonierten, Sulfongruppen enthaltenden Polymere
b) 25 bis 94,95 Gew.-% Wasser
c) 5 - 50 Gew.-% Tenside
c) 0 - 5 Gew.-% eines weiteren Konditioniermittels
d) 0 -10 Gew.-% weitere kosmetische Bestandteile

In den Shampooformulierungen können alle in Shampoos üblicherweise eingesetzten anionischen, neutralen, amphoteren oder kationischen Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylglykolalkoxylate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Geeignet sind zum Beispiel Natriumlaurylsulfat, Ammoniumlaurylsulfat, Natriumlaurylethersulfat, Ammoniumlaurylethersulfat, Natriumlauroylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mol auf ein Mol Alkohol. Ferner sind Alkylaminoxide, Mono- oder Dialkylalkanolamide, Fettsäureester von Polyethylenglykolen, ethoxylierte Fettsäureamide, Alkylpolyglykoside, Alkylglykolalkoxylate und -diglykolalkoxylate oder Sorbitanetherester geeignet.

Geeignete anionische Tenside für die erfindungsgemäßen kosmetischen Zubereitungen sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate, Alkylglykolalkoxylate und -diglykolalkoxylate und Alkylethercarboxylate können zwischen 1 bis 10 Ethylenoxid oder Propylenoxid-Einheiten, bevorzugt 1 bis 3 Ethylenoxideinheiten im Molekül aufweisen. Geeignet sind zum Beispiel Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Außerdem können die Mittel übliche kationische Tenside enthalten, wie z.B. quaternäre Ammoniumverbindungen, beispielsweise Cetyltrimethylammoniumchlorid.

Werden die erfindungsgemäßen (Meth)Acrylatpolymerisate in Shampooformulierungen eingesetzt, so enthalten diese üblicherweise anionische Tenside als Basistenside und amphotere und nichtionische Tenside als Cotenside.

Die kosmetischen Zubereitungen enthalten üblicherweise 2 bis 50 Gew.-% Tenside, bevorzugt 5 bis 40 Gew.%, besonders bevorzugt 8 bis 30 Gew-%.

Außerdem können die Shampooformulierungen übliche kationische Tenside enthalten, wie z.B. quaternäre Ammoniumverbindungen, beispielsweise Cetyltrimethylammoniumchlorid oder -bromid (INCI Cetrimoniumchloride oder -bromide), Hydroxyethylcetyldimonium-phosphat (INCI Quaternium-44), INCI Cocotrimoniummethosulfate, INCI Quaternium-52.

In den Shampooformulierungen können zur Erzielung bestimmter Effekte übliche Konditioniermittel in Kombination mit den erfindungsgemäßen Polymerisaten eingesetzt werden. Hierzu zählen beispielsweise kationische Polymere mit der Bezeichnung Polyquaternium nach INCI, z.B. Copolymere aus Vinylpyrrolidon/N-Vinylimidazoliumsalzen (Luviquat® FC, Luviquat® HM, Luviquat® MS, Luviquat® Care, INCI: Polyquaternium-16, Polyquaternium-44), Copolymere aus N-Vinylpyrrolidon/Dimethylaminoethylmethacrylat, quaternisiert mit Diethylsulfat (Luviquat® PQ 11, INCI: Polyquaternium-11), Copolymere aus N-Vinylcaprolactam N-Vinylpyrrolidon/N-Vinylimidazoliumsalzen (Luviquat® Hold, INCI: Polyquaternium-46); Copolymere aus Acrylamid und Dimethyldiallylammoniumchlorid (Polyquaternium-7), kationische Cellulosederivate (Polyquaternium-4, -10). Ferner können kationische Stärkederivate (INCI: Starch Hydroxypropytrimonium Chloride, Corn Starch Modified), kationische Guarderivate (INCI: Hydroxypropyl Guar Hydroxypropyltrimonium Chloride), kationische Sonnenblumenöl-Derivate (INCI: Sunflowerseedamidopropyl Hydroxyethyldimonium Chloride), Copolymere aus Acrylsäure, Acrylamid und Methacrylamidopropyltrimoniumchlorid (INCI: Polyquaternium-53), Polyquaternium-32, Polyquaternium-28 und andere eingesetzt werden. Ferner können Eiweißhydrolysate verwendet werden, sowie konditionierende Substanzen auf Basis von Silikonverbindungen, beispielsweise Polyalkylsiloxane, Polyarylsiloxane, Polyarylalkylsiloxane, Polyethersiloxane oder Silikonharze. Weitere geeignete Silikonverbindungen sind Dimethicone, Dimethicone-Derivate oder Dimethicon Copolyole (CTFA) und aminofunktionelle Silikonverbindungen wie Amodimethicone (CTFA).

### Emulgatoren

Als Emulgatoren für die erfindungsgemäßen kosmetischen Zubereitungen kommen beispielsweise nichtionogene Tenside aus mindestens einer der folgenden Gruppen in Frage:
(1) Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atömen und an Alkylphenole mit 8 bis 15 C-Atomen in der Alkylgruppe;
(2) C12/18-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C₆/₁₂-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(9) Mono-, Di- und Trialkylphosphate sowie Mono-, Di- und/oder Tri-PEG-alkylphosphate und deren Salze;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß DE-PS 1165574 und/oder Mischester von Fettsäuren mit 6 bis 22 Kohlenstoffatomen, Methylglycose und Polyolen, vorzugsweise Glycerin oder Polyglycerin sowie
(13) Polyalkylenglykole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C₁₂- bis C₁₈-Fettsäuremono- und -diester von Anlagerungsprodukten von Ethylenoxid an Glycerin sind aus DE-PS 2024051 als Rückfettungsmittel für kosmetische Zubereitungen bekannt. C₈ bis C₁₈-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidesters gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und/oder eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxy-methylglycinat.

Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C₈ bis C₁₈-Alkyl- oder -Acylgruppe im Molekül mindestens eine freie Aminogruppe und mindestens eine -COOH- und/oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂ bis C₁₈-Acylsarcosin.

Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

### Konservierungsmittel

Als Konservierungsmittel für die erfindungsgemäßen kosmetischen Zubereitungen eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen.

### Parfümöle

Gegebenenfalls können die kosmetischen Zubereitungen Parfümöle enthalten. Als Parfümöle seien beispielsweise Gemische aus natürlichen und synthetischen Riechstoffen genannt. Natürliche Riechstoffe sind Extrakte von Blüten (Lilie, Lavendel, Rose, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orange), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, 4-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonat, zu den Ketonen z.B. die Jonone, cc-Isomethylionen und Methylcedrylketon, zu den Alkoholen Anethof, Citronellol, Eugenol, Isoeugenol, Geraniol, Linalool, Phenylethylalkohol und Terioneol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzeöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene® Forte, Ambroxan®, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix® Coeur, Iso-E-Super®, Fixolide® NP, Evernyl®, Iraldein gamma, Phenylessigsäure, Geranylacetat, Benzylacetat, Rosenoxid, Romillat, Irotyl und Floramat allein oder in Mischungen eingesetzt.

### Antioxidantien

Die Antioxidantien für die erfindungsgemäßen kosmetischen Zubereitungen sind in der Regel an sich bekannte Verbindungen. Vorteilhaft werden die Antioxidantien ausgewählt aus den Gruppen der Carotinoide, Carotine (z.B. α-Carotin, β-Carotin, Lycopin) und deren Derivate, Chlorogensäure und deren Derivate, Liponsäure und deren Derivate (z.B. Dihydroliponsäure), ferner (Metall)Chelatoren, EDTA, EGTA und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat), Butylhydroxytoluol, Butylhydroxyanisol, sowie weitere üblicherweise in kosmetischen Zubereitungen verwendete Antioxidantien.

Die Menge der vorgenannten Antioxidantien in den fertigen Zubereitungen beträgt z.B. 0,001 bis 30 Gew.-%, vorzugsweise 0,01 bis 10 Gew.-% und insbesondere 1 bis 5 Gew.-%.

In den erfindungsgemäßen kosmetischen Zubereitungen können die Antioxidatien vorteilhaft zusammen mit Peroxidzersetzern eingesetzt werden. Insbesondere können synergistisch wirksame Kombinationen von Antioxidantien mit Peroxidzersetzern eingesetzt werden. Bevorzugt werden Antioxidantien in synergistisch wirksamer Kombination mit peroxidzersetzenden Bor-Verbindungen wie sie in WO 03/059312 beschrieben sind, eingesetzt.

### UV-Lichtschutz

Die in kosmetischen Zubereitungen eingesetzten Lichtschutzfilter haben die Aufgabe, schädigende Einflüsse des Sonnenlichts auf die menschliche Haut zu verhindern oder zumindest in ihren Auswirkungen zu reduzieren. Daneben dienen diese Lichtschutzfilter aber auch dem Schutz weiterer Inhaltsstoffe vor Zerstörung oder Abbau durch UV-Strahlung. In haarkosmetischen Formulierungen soll eine Schädigung der Keratinfaser durch UV-Strahlen vermindert werden.

Das an die Erdoberfläche gelangende Sonnenlicht hat einen Anteil an UV-B- (280 bis 320 nm) und an UV-A-Strahlung (320 bis 400 nm), welche sich direkt an den Bereich des sichtbaren Lichtes anschließen. Der Einfluß auf die menschliche Haut macht sich besonders bei der UV-B-Strahlung durch Sonnenbrand bemerkbar.

Als ein Maximum der Erythemwirksamkeit des Sonnenlichtes wird der engere Bereich um 308 nm angegeben.

Zum Schutz gegen UV-B-Strahlung sind zahlreiche Verbindungen bekannt, bei denen es sich u.a. um Derivate des 3-Benzylidencamphers, der 4-Aminobenzoesäure, der Zimtsäure, der Salicylsäure, des Benzophenons sowie des 2-Phenylbenzimidazols handelt.

Auch für den Bereich zwischen etwa 320 nm und etwa 400 nm, des sogenannten UV-A-Bereich, ist es wichtig, Filtersubstanzen zur Verfügung zu haben, da dessen Strahlen Reaktionen bei lichtempfindlicher Haut hervorrufen können. Es ist erwiesen, daß UV-A-Strahlung zu einer Schädigung der elastischen und kollagenen Fasern des Bindegewebes führt, was die Haut vorzeitig altern läßt, und daß sie als Ursache zahlreicher phototoxischer und photoallergischer Reaktionen zu sehen ist. Der schädigende Einfluß der UV-B-Strahlung kann durch UV-A-Strahlung verstärkt werden.

Als UV-Lichtschutzfilter können öllösliche organische UV-A-Filter und/oder UV-B-Filter und/oder wasserlösliche organische UV-A-Filter und/oder UV-B-Filter eingesetzt werden. Die Gesamtmenge der UV-Lichtschutzfilter liegt in der Regel bei 0,1 Gew.-% bis 30 Gew.-%, vorzugsweise 0,5 bis 15 Gew.-%, insbesondere 1 bis 10 Gew.-%, bezogen auf das Gesamtgewicht der Zubereitungen.

Vorteilhafterweise werden die UV-Lichtschutzfilter so gewählt, daß die Zubereitungen die Haut vor dem gesamten Bereich der ultravioletten Strahlung schützen.

### Beispielsweise sind als UV-Lichtschutzfilter zu nennen:

| Nr. | Stoff |
|---|---|
| 1 | 4-Aminobenzoesäure |
| 2 | 3-(4'Trimethylammonium)-benzylidenbornan-2-on-methylsuffat |
| 3 | 3,3,5- Trimethyl-cyclohexyl-salicylat (Homosalatum) |
| 4 | 2-Hydroxy-4-methoxy-benzophenon(Oxybenzonum) |
| 5 | 2-Phenylbenzimidazol-5-sulfonsäure und ihre Kalium-, Natrium- u. Triethanolaminsalze |
| 6 | 3,3'-(1,4-Phenylendimethin)-bis(7,7-dimethyl-2-oxobicyclo[2.2.1]heptan-1-methansulfonsäure) und ihre Salze |
| 7 | 4-Bis(polyethoxy)amino-benzoesäurepolyethoxy-ethylester |
| 8 | 4-Dimethylamino-benzoesäure-2-ethylhexylester |
| 9 | Salicylsäure-2-ethylhexylester |
| 10 | 4-Methoxy-zimtsäure-2-isoamylester |
| 11 | 4-Methoxy-zimtsäure-2-ethylhexylester |
| 12 | 2-Hydroxy-4-methoxy-benzophenon-5-sulfonsäure-(Sulisobenzonum) und das Natriumsalz |
| 13 | 3-(4'-Sulfo)benzyliden-bornan-2-on und Salze |
| 14 | 3-Benzylidenbornan-2-on |
| 15 | 1-(4'-Isopropylphenyl)-3-phenylpropan-1,3-dion |
| 16 | 4-Isopropylbenzylsalicylat |
| 17 | 2,4,6-Trianilin-(o-carbo-2'-ethylhexyl-1'-oxy)-1,3,5-triazin |
| 18 | 3-Imidazol-4-yl-acrylsäure und ihr Ethylester |
| 19 | Menthyl-o-aminobenzoate oder:5-Methyl-2-(1-methylethyl)-2-aminobenzoate |
| 20 | Glyceryl p-aminobenzoat oder 4-Aminobenzoesäure-1-glyceryl-ester |
| 21 | 2,2'-Dihydroxy-4-methoxybenzophenon (Dioxybenzone) |
| 22 | 2-Hydroxy-4-methoxy-4-methylbenzophenon (Mexenon) |
| 23 | Triethanolamin Salicylat |
| 24 | Dimethoicyphenylglyoxalsäure oder:3,4-dimethoxy-phenyl-glyoxal-saures Natrium |
| 25 | 3-(4'Sulfo)benzyliden-bornan-2-on und seine Salze |
| 26 | 2,2',4,4'-Tetrahydroxybenzophenon |
| 27 | 2,2'-Methylen-bis-[6(2H-benzotriazol-2-yl)-4-(1,1,3,3-tetramethylbutyl)phenol] |
| 28 | 2,2'-(1,4-Phenylen)-bis-1H-benzimidazol-4,6-disulfonsäure, Na-Salz |
| 29 | 2,4-bis-[4-(2-Ethylhexyloxy)-2-hydroxy]phenyl-6-(4-methoxyphenyl)-(1,3,5)-triazin |
| 30 | 3-(4-Methylbenzyliden)-campher |
| 31 | 4-Bis(polyethoxy)paraaminobenzoesäurepolyethoxyethylester |
| 32 | 2,4-Dihydroxybenzophenon |
| 33 | 2,2'-Dihydroxy-4,4'-dimethoxybenzophenon-5,5'-dinatriumsulfonat |

### Farbstoffe

Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S. 81-106, zusammengestellt sind. Diese Farbstoffe werden üblicherweise in Konzentrationen von 0,001 bis 0,1 Gew.-%, bezogen auf die gesamte Mischung, eingesetzt.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Fette

Typische Beispiele für Fette sind Glyceride, als Wachse kommen u.a. Bienenwachs, Carnaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als Stabilisatoren können Metallsalze von Fettsäuren, wie z.B. Magnesium-, Calcium-, Aluminium- und/oder Zinkstearat bzw. -ricinoleat eingesetzt werden.

Geeignete Silikonverbindungen sind beispielsweise Dimethylpolysiloxane, Methylphenylpolysiloxane, cyclische Silikone sowie amino-, fettsäure-, alkohol-, polyether-, epoxy-, fluor-, glykosid- und/oder alkylmodifizierte Silikonverbindungen, die bei Raumtemperatur sowohl flüssig als auch harzförmig vorliegen können.

### Pharmazeutische Zubereitungen

Ein Gegenstand der Erfindung betrifft pharmazeutische Mittel enthaltend sulfonierte, Sulfongruppen enthaltende Polymere und pharmazeutisch übliche Wirkstoffe und Zusatzstoffe.

Feste pharmazeutische Darreichungsformen wie Tabletten, Kapseln, Pellets, Granulate, Kristalle etc. werden aus sehr unterschiedlichen Gründen gecoatet, d.h. mit einem Filmüberzug versehen. So kann beispielsweise ein schlechter Geruch oder Geschmack maskiert sowie die Schluckbarkeit verbessert werden. Die Stabilität des Wirkstoffes kann durch das Coating erhöht werden, indem weniger Wasserdampf und Sauerstoff an das Tabletteninnere gelangt. Die Darreichungsformen sehen besser aus und können durch die Einarbeitung von Farbstoffen besser unterschieden werden. Darüber hinaus lässt sich insbesondere die Freisetzungsgeschwindigkeit des Wirkstoffes durch den Filmüberzug einstellen.

Generell unterscheidet man Instant-Release-Formen und Retard- bzw. Slow-Release-Formen.

Bei Instant-Release-Formen sollen der Zerfall der Tablette und die Freisetzung des Wirkstoffs aus der Darreichungsform nach Möglichkeit nicht durch das Coating beeinflußt werden, deshalb muss sich der Filmüberzug schnell im Magensaft auflösen. Daneben muss er über gute Filmeigenschaften verfügen. Die Zugfestigkeit und die Reißdehnung sollten hoch sein, damit der Filmüberzug mechanischen Einwirkungen standhält, wie sie bei der pharmazeutischen Verarbeitung - insbesondere der Konfektionierung - und auch während des Versandes bzw. der Lagerung auftreten. Ein häufig eingesetztes Produkt für das Coaten von Instant-Release-Tabletten ist Hydroxypropylmethylcellulose (HPMC). Hydroxypropylmethylcellulose weist in wässriger Lösung bei zunehmender Konzentration einen steilen Viskositätsanstieg auf. Ein ähnliches Verhalten zeigt auch Hydroxypropylcellulose (HPC).

Da die Filmbildnerlösung beim Coaten von Tabletten fein zerstäubt werden muss und die gebildeten Tröpfchen die Oberfläche der Tabletten gut benetzen und auch gut spreiten müssen, darf die Viskosität eine gewisse Grenze (zwischen 150 und 250 mPas), die abhängig ist von der Art der Sprühdüse und des Gerätes, nicht überschreiten. Deshalb können im Falle von HPMC nur verhältnismäßig niedrige Filmbildnerkonzentrationen eingesetzt werden.

Als Empfehlung für die Konzentration von Pharmacoat® 606 (Fa. Shin-etsu) werden in der Literatur 5 bis 7 Gew.-% angegeben (Pharmaceutical Coating Technology, Hrsg. Graham Cole, Taylor und Francis Ltd. 1995 und Technische Merkblätter der Hersteller). Diese geringe Sprühkonzentrationen bedingen relativ lange Verarbeitungszeiten und damit hohe Kosten.

Darüber hinaus zeigt Hydroxypropylmethylcellulose weitere Nachteile u.a. im Benetzungsverhalten, im Pigmentbindevermögen, in den mechanischen Eigenschaften der Filme, in der Hygroskopizität sowie in der Permeabilität gegenüber Wasserdampf und Sauerstoff, in der Auflösungsgeschwindigkeit und in der Zerfallszeitdifferenz zwischen Filmtabletten und Kern.

Die geringe Elastizität der Filme aus Hydroxypropylmethylcellulose führt häufig dazu, daß die Filmtabletten bei feuchter Lagerung infolge der Quellung des Kerns aufreißen. Auch der Einsatz von Weichmachern ergibt keine nennenswerten Verbesserungen dieses Problems. Er kann vielmehr zu klebrigen Filmen und durch Migration zu Veränderungen der Tabletteneigenschaften führen.

Orale Arzneiformen mit einer Arzneistofffreigabe über einen längeren Zeitraum mit dem Ziel der Wirkungsverlängerung der aktiven Komponente (allgemein Retardarzneiformen) gewinnen zunehmend an Bedeutung. Mit ihr sind eine verbesserte Patientencompliance durch eine reduzierte Einnahmefrequenz, eine Verringerung von Nebenwirkungen durch Vermeidung von Plasmaspitzen, gleichmäßigere Blutspiegel des Arzneistoffs sowie die Vermeidung von lokalen Irritationen vorteilhaft verbunden. Neben der Formulierung von arzneistoffhaltigen Kernen, die mit einem wasserunlöslichen aber semipermeablen bzw. porenhaltigen Film überzogen wurden, durch die der Arzneistoff diffundiert, kann die Steuerung und Verlängerung der Freisetzung durch die Einbettung des Arzneistoffes in Matrices erreicht werden. Weiterhin ist der Einsatz von lonenaustauscherharzen und therapeutischen Systemen (z.B. OROS) möglich.

Besonders die Einbettung des Arzneistoffs in Hydrokolloidmatrices bietet die Vorteile einer einfachen und preiswerten Herstellung und einer hohen Arzneimittelsicherheit, da Dose Dumping Effekte nicht auftreten können. Die hierfür in der Regel eingesetzten Hilfsstoffe wie Hydroxypropylmethylcellulose (HPMC), Hydroxypropylcellulose, Alginsäure bzw. Alginate sowie Xanthan besitzen Anwendungsnachteile. Hier sind zu nennen: Mangelnde Fließeigenschaften, die eine Direkttablettierung erschweren, eine Abhängigkeit der Arzneistofffreigabe von der Osmolarität (Salzgehalt) und vom pH-Wert des Freisetzungsmediums. Dies gilt ebenso für HPMC wie für Hydroxypropylcellulose, Xanthan und Alginate. Die Verwendung von Xanthan führt ferner zu Tabletten mit geringer Härte, die Direkttablettierung von Alginaten resultiert in Presslingen mit nur noch geringen retardierenden Eigenschaften (max. 8 h). Die natürlichen Quellstoffe (z.B. Alginate) besitzen insgesamt eine starke Chargenvariabilität.

Bindemittel werden in pharmazeutischen Darreichungsformen eingesetzt, um die Verarbeitbarkeit und die mechanische Festigkeit zu erhöhen. Sie werden üblicherweise in Tabletten, Granulaten und Pellets eingesetzt und führen zu verbesserter Fließfähigkeit, höherer Bruchfestigkeit und geringerer Friabilität.

Die derzeit verwendeten Bindemittel wie Maltodextrin oder Polyvinylpyrrolidone führen häufig zu nicht zufriedenstellenden Bruchfestigkeiten und Friabilitäten. Andere Bindemittel wie Stärkekleister und Hydroxypropylmethylcellulose (HPMC) lassen sich aufgrund ihrer hohen Viskosität nur niedrigkonzentriert einsetzen.

Weiterhin werden filmbildende Hilfsstoffe in Lösungen und Sprays eingesetzt, die auf der Haut oder nicht oralen Schleimhaut aufgebracht oder auch dem Körper systemisch zugeführt werden. Beispiele hierfür sind Zubereitungen für die Wundbehandlung, Sprayverbände aber auch Zubereitungen zur Applikation auf intakter Haut bzw. nicht oraler Schleimhaut. Dabei wird die Haut durch einen Film geschützt und die Wirkstoffe können in bzw. durch die Haut dringen.

Bei transdermalen therapeutischen Systemen und bei Wundpflastern ist ebenfalls wie bei den oben genannten Darreichungsformen eine ausreichende Flexibilität erforderlich, die die derzeit zur Verfügung stehenden Produkte nicht aufweisen. Der Einsatz von möglichen Weichmachern zur Erreichung der notwendigen Flexibilität ist aus toxikologischen und pharmakologischen Gründen nicht erwünscht.

Eine Aufgabe der vorliegenden Erfindung bestand darin, wasserlösliche oder wasserdispergierbare Polymerisate als Überzugsmittel, Bindemittel und/oder Filmbildner in pharmazeutischen Zubereitungen bereitzustellen, die die oben genannten Nachteile nicht aufweisen.

Überraschenderweise wurde gefunden, daß sich sulfonierte, Sulfongruppen enthaltende Polymere zur Verwendung in pharmazeutischen Zubereitungen eignen.

Sie eignen sich insbesondere als Überzugsmittel, Bindemittel, Filmbildner und auch als Matrix für die Wirkstofffreisetzung in pharmazeutischen Zubereitungen.

Die erfindungsgemäßen Polymerisate können in einer Vielzahl von pharmazeutischen Zubereitungen eingesetzt werden.

Beispielsweise können genannt werden als überzogene Zubereitungen Filmtabletten, Filmmikrotabletten, Dragees, überzogene Pastillen, Kapseln, Kristalle, Granulate oder Pellets.

Bei bindemittelhaltige Zubereitungen handelt es sich um beispielsweise Tabletten, Mikrotabletten, Kerne, Granulate oder Pellets.

Weiterhin können die sulfonierten, Sulfongruppen enthaltenden Polymere zur Herstellung von Lösungen und Sprays verwendet werden, die, auf Haut oder nicht orale Schleimhaut aufgebracht, einen Film ausbilden.

Beispiele hierfür sind Sprühverbände für Wunden, Desinfektionssprays oder Lösungen mit Mycostatica. Auch der Einsatz bei transdermalen therapeutischen Systemen möglich.

Die erfindungsgemäß verwendeten sulfonierten, Sulfongruppen enthaltenden Polymere benetzen leicht lipophile Oberflächen und besitzen hervorragende Schutzkolloideigenschaften. Eingearbeitet in Suspensionen und Emulsionen lagern sie sich an die Teilchen der dispersen Phase und stabilisieren diese. Sie können daher als Benetzungshilfsmittel und Stabilisatoren in dispersen Systemen verwendet werden.

Durch Wechselwirkung mit schwer wasserlöslichen Arzneistoffen verbessern sie deren Löslichkeit und Lösungsgeschwindigkeit, wodurch Resorbierbarkeit und Bioverfügbarkeit der Arzneistoffe verbessert werden. Diese vorteilhafte Wirkung zeigt sich beispielsweise bei den Darreichungsformen, bei denen der Wirkstoff nicht gelöst vorliegt, wie z.B. Tabletten, Granulate, Suspensionen etc..

Die erfindungsgemäß verwendeten Polymerisate können gegebenenfalls auch in Kombination mit anderen Hilfsstoffen zusammen mit Wirkstoffen zu Polymer-Wirkstoffschmelzen verarbeitet werden, die entweder zu Arzneistoffen extrudiert und kalandriert werden oder nach der Extrusion zu Granulaten oder Pulvern zerteilt werden und erst anschließend in Arzneiformen verarbeitet werden, beispielsweise zu Tabletten verpresst werden. Dabei bringen sulfonierten, Sulfongruppen enthaltenden Polymere die bereits oben aufgeführten Eigenschaften in die Zubereitungen ein.

In verschiedenen pharmazeutischen Zubereitungen können sulfonierte, Sulfongruppen enthaltende Polymere folgende Funktionen hervorragend erfüllen:
Dispergierhilfsstoff, Suspendierhilfsstoff, Benetzungsmittel, Solubilisator für schwerlösliche Arzneistoffe, Emulgator, Kristallisationsinhibitor, Anticakinghilfsstoff, Schutzkolloid, Bioadhäsivum zur Verlängerung und Intensivierung des Kontaktes mit der nicht oralen Schleimhaut, Spreithilfsmittel, Viskositätsregulator, Hilfsstoff zur Herstellung von festen Lösungen mit Arzneistoffen, Hilfsstoff zur Einstellung der Wirkstofffreisetzung in Retardformulierungen.

Die Löslichkeit der sulfonierten, Sulfongruppen enthaltenden Polymere kann durch geeignete Wahl des Sulfonierungsgrades beliebig eingestellt werden.

So können in Wasser nicht oder nur gering lösliche, aber dispergierbare sulfonierte, Sulfongruppen enthaltende Polymere auch als Retardierungspolymere verwendet werden.

Bei der Verwendung zur Herstellung von Suppositorien und Vaginalglobuli gewährleisten die sulfonierten, Sulfongruppen enthaltenden Polymere einerseits die Flexibilität der Darreichungsform und fördern andererseits den Zerfall und die Wirkstoffauflösung und sie kleiden die nicht orale Schleimhaut mit einem wirkstoffhaltigen Film aus, der die Resorption verstärkt.

Tabletten quellen in Abhängigkeit von den verwendeten Hilfs- und Wirkstoffen, der Lagerzeit und den Lagerbedingungen, wie Temperatur und Feuchtigkeit, unterschiedlich stark. Ein starrer Filmüberzug erleidet bei Quellung des Kernes Risse. Deshalb ist die Elastizität von Filmbildnern eine wichtige Größe.

Die gegebenenfalls neutralisierten sulfonierten, Sulfongruppen enthaltenden Polymere können in reiner Form oder aber zusammen mit den üblichen Hilfsstoffen auf den wirkstoffhaltigen Kern appliziert werden. Übliche Hilfsstoffe sind z.B. Farbpigmente zur Einfärbung, Weißpigmente, wie Titandioxid, zur Erhöhung der Deckkraft, Talkum und Siliciumdioxid als Antiklebemittel, Polyethylenglykole, Glycerin, Propylenglykol, Triacetin, Triethylcitrat als Weichmacher und verschiedene oberflächenaktive Stoffe, wie Natriumlaurylsulfat, Polysorbat 80, Pluronics und Cremophore, zur Verbesserung des Benetzungsverhaltens. Die exemplarisch genannten Stoffe stellen keine Begrenzung dar. Es können alle bekanntermaßen für magensaftlösliche Filmüberzüge geeigneten Zusatzstoffe verwendet werden.

Als Überzugsverfahren lassen sich die gebräuchlichen Verfahren, wie das Coaten in der Wirbelschicht oder im Horizontaltrommelcoater, das Tauchschwertverfahren und das Kesselcoatingverfahren, anwenden. Neben der Anwendung auf Tabletten können die sulfonierten, Sulfongruppen enthaltenden Polymere auch für das Coating von anderen pharmazeutischen Zubereitungen, wie Granulaten, Pellets, Kristallen oder Kapseln, eingesetzt werden. Die neuen Überzugsmittel werden wie üblich in einer Stärke von 5 bis 200 *µ*m, vorzugsweise 10 bis 100 *µ*m, aufgetragen.

Bei der Verwendung als Bindemittel unterscheidet man je nach Verarbeitungsverfahren zwischen Feucht- und Trockenbindemittel. Letztere werden u.a. bei der Direkttablettierung und bei der Trockengranulation bzw. Kompaktierung verwendet. Hierbei wird das Bindemittel mit dem Wirkstoff und gegebenenfalls weiteren Hilfsstoffen vermischt und anschließend direkttablettiert oder granuliert bzw. kompaktiert.

Im Gegensatz dazu wird bei der Feuchtgranulation die Wirkstoff-Hilfsstoffmischung mit einer Lösung des Bindemittels in Wasser oder einem organischen Lösungsmittel befeuchtet, die feuchte Masse durch ein Sieb gegeben und anschließend getrocknet. Befeuchtung und Trocknung können dabei auch parallel ablaufen, wie z.B. in der Wirbelschichtgranulation.

Für eine optimale Verarbeitung soll das Bindemittel in Lösung niedrigviskos sein, da viskose Lösungen zu inhomogenen Granulaten führen.

Ein Bindemittel soll zu gleichmäßigen, harten, abriebsstabilen Granulaten bzw. Tabletten führen. Insbesondere bei Tabletten kommt der Bruchfestigkeit besondere Bedeutung zu, da sich viele Wirkstoffe schlecht verpressen lassen und somit Tabletten mit unzureichender mechanischer Stabilität ergeben.

Weiterhin soll der Zerfall der Arzneiformen sowie die Freisetzungsgeschwindigkeit der Wirkstoffe durch das Bindemittel nicht nennenswert nachteilig beeinflusst werden.

Die gängigsten Bindemittel sind beispielsweise Polyvinylpyrrolidon, Vinylacetat-Vinylpyrrolidon Copolymere, Gelatine, Stärkekleister, Maltodextrine, hydroxyalkylierte bzw. carboxyalkylierte Cellulosederivate, wie Hydroxypropylmethylcellulose, Methylcellulose, Natriumcarboxymethylcellulose sowie natürliche Gummisorten, wie beispielsweise Gummi Arabicum, Pektin oder Alginat.

Viele dieser Bindemittel weisen in Lösung eine hohe Viskosität auf und sind schlecht verarbeitbar. Durch die hohe Viskosität werden die zu granulierenden Pulverteilchen schlecht und ungleichmäßig benetzt, so dass daraus eine zu geringe Granulatfestigkeit und eine ungünstige Korngrößenverteilung resultieren.

Viele Bindemittel sind zudem hygroskopisch und quellen bei Wasseraufnahme. Dadurch können sich die Granulat- und Tabletteneigenschaften dramatisch verändern.

Überraschenderweise wurde nun gefunden, dass die sulfonierten, Sulfongruppen enthaltenden Polymere über ausgezeichnete Bindemittelwirkungen verfügen und zudem in Konzentrationsbereichen von 0,5 bis 20 Gew.-%, bevorzugt 1 bis 10 Gew.-% der Gesamtmenge der Formulierung den Zerfall nicht nennenswert beeinflussen. Aufgrund des guten Benetzungsverhaltens kann sich zudem die Freisetzung von schlecht löslichen Wirkstoffen verbessern. Hervorzuheben ist auch die vergleichsweise geringe Viskosität der Polymerlösungen.

Bei Bedarf können selbstverständlich die pharmazeutisch akzeptablen, für haut- und/oder haarkosmetische Zubereitungen geeigneten Zusatzstoffe und Hilfsmittel in den pharmazeutischen Zubereitungen verwendet werden.

Mit den Polymerisaten als Bindemittel ergeben sich mechanisch außerordentlich stabile und auch über lange Lagerzeiten stabile Granulate bzw. Tabletten.

### Anwendungstechnische Beispiele

### Beispiel 1:

| VOC 80-Aerosol-Haarspray | [%] |
|---|---|
| Polymer Bsp. 1 EP-A 045 194 (10 %ige wässrige Lösung) | 12,00 |
| Wasser | 8,00 |
| Dimethylether | 40,00 |
| Ethanol | 40,00 |
| weiterer Zusatz: Silikon, Parfüm, Entschäumer | |

Das Beispiel lässt sich mit den Polymeren aus den Beispielen 2, 5, 6 und 7 der EP-A 045 194 wiederholen. Es wird jeweils ein VOC 80-Aerosol-Haarspray mit guten Eigenschaften erhalten.

### Beispiel 2:

| VOC 55-Aerosol-Haarspray | [%] |
|---|---|
| Polymer Bsp. 1 EP-A 045 194 (10 %ige wässrige Lösung) | 12,00 |
| Wasser | 33,00 |
| Dimethylether | 40,00 |
| Ethanol | 15,00 |
| weiterer Zusatz: Silikon, Parfüm, Entschäumer | |

Das Beispiel lässt sich mit den Polymeren aus den Beispielen 2, 5, 6 und 7 der EP-A 045 194 wiederholen. Es wird jeweils ein VOC 55-Aerosol-Haarspray mit guten Eigenschaften erhalten.

### Beispiel 3:

| VOC 55-Aerosol-Haarspray | [%] |
|---|---|
| Polymer Bsp. 1 EP-A 045 194 (10 %ige wässrige Lösung) | 10,00 |
| Ultrahold Strong (Fa. BASF) | 1,00 |
| Wasser | 34,00 |
| Dimethylether | 40,00 |
| Ethanol | 15,00 |
| + AMP | auf pH 8,3 |
| weiterer Zusatz: Silikon, Parfüm, Entschäumer... | |

Das Beispiel lässt sich mit den Polymeren aus den Beispielen 2, 5, 6 und 7 der EP-A 045 194 wiederholen. Es wird jeweils ein VOC 55-Aerosol-Haarspray mit guten Eigenschaften erhalten.

### Beispiel 4:

| VOC 55-Aerosol-Haarspray | [%] |
|---|---|
| Polymer Bsp. 1 EP-A 045 194 (10 %ige wässrige Lösung) | 8,00 |
| Stepanhold R-1^{°)}(Fa. Stepan Chemical Co.) | 1,00 |
| Wasser | 36,00 |
| Dimethylether | 40,00 |
| Ethanol | 15,00 |
| + AMP | auf pH 8,3 |
| weiterer Zusatz: Silikon, Parfüm, Entschäumer ... | |

| | |
|---|---|
| ^{*)} Stepanhold R-1 = Poly(Vinylpyrrolidon/Ethylmethacrylat/Methacrylsäure) | |

Das Beispiel lässt sich mit den Polymeren aus den Beispielen 2, 5, 6 und 7 der EP-A 045 194 wiederholen. Es wird jeweils ein VOC 55-Aerosol-Haarspray mit guten Eigenschaften erhalten.

### Beispiel 5:

| VOC 55-Handpumpenspray | [%] |
|---|---|
| Polymer Bsp. 1 EP-A 045 194 (10 %ige wässrige Lösung) | 12,00 |
| Wasser | 33,00 |
| Ethanol | 55,00 |
| weiterer Zusatz: Silikon, Parfüm, Entschäumer ... | |

Das Beispiel lässt sich mit den Polymeren aus den Beispielen 2, 5, 6 und 7 der EP-A 045 194 wiederholen. Es wird jeweils ein VOC 55-Handpumpenspray mit guten Eigenschaften erhalten.

### Beispiel 6:

| Wässriges Handpumpenspray | [%] |
|---|---|
| Polymer Bsp. 1 EP-A 045 194 (10 %ige wässrige Lösung) | 10,00 |
| Luviset Clear ^{*)} (10 %ige Lösung) | 5,00 |
| Wasser | 85,00 |
| weiterer Zusatz: wasserlösliches Silikon, Parfüm, Entschäumer ... | |

| | |
|---|---|
| ^{*)} Luviset Clear:Poly(Vinylpyrrolidon/Methacrylsäureamid/Vinylimidazol), Fa. BASF | |

Das Beispiel lässt sich mit den Polymeren aus den Beispielen 2, 5, 6 und 7 der EP-A 045 194 wiederholen. Es wird jeweils ein wässriges Handpumpenspray mit guten Eigenschaften erhalten.

### Beispiel 7:

| Schaumconditioner | [%] |
|---|---|
| Polymer Bsp. 1 EP-A 045 194 (10 %ige wässrige Lösung) | 20,00 |
| Cremophor A 25 (Ceteareth 25/Fa. BASF) | 0,2 |
| Comperlan KD (Coamide DEA/Fa. Henkel) | 0,1 |
| Wasser | 69,7 |
| Propan/Butan | 10,0 |
| weiterer Zusatz: Parfüm, Konservierungsmittel .... | |

Herstellung: Einwiegen und unter Rühren lösen. Abfüllen und Treibgas zusetzen.

Das Beispiel lässt sich mit den Polymeren aus den Beispielen 2, 5, 6 und 7 der EP-A 045 194 wiederholen. Es wird jeweils ein Schaumconditioner mit guten Eigenschaften erhalten.

### Beispiel 8:

| Haargel mit Aculyn 28: | [%] |
|---|---|
| Phase 1: | |
| Polymer Bsp. 1 EP-A 045 194 (10 %ige wässrige Lösung) | 12,00 |
| Wasser, dest. | 37,00 |
| Aminomethylpropanol (38 %ige Lösung) | 1,0 |
| weiterer Zusatz: Konservierungsmittel, lösliches ethoxiliertes Silikon, Parfüm ... | |

| Phase 2: | |
|---|---|
| Aculyn 28 (1 %ige wässrige Suspension) | 50,00 |

### Herstellung:

Die Phasen 1 und 2 werden getrennt eingewogen und homogenisiert. Dann wird Phase 2 langsam in Phase 1 eingerührt. Es bildet sich ein im Wesentlichen klares, stabiles Gel.

Das Beispiel lässt sich mit den Polymeren aus den Beispielen 2, 5, 6 und 7 der EP-A 045 194 wiederholen. Es wird jeweils ein Haargel mit Aculyn 28 mit guten Eigenschaften erhalten.

### Beispiel 9:

| Haargel mit Hydroxyethylcellulose: | [%] |
|---|---|
| Phase 1: | |
| Polymer Bsp. 1 EP-A 045 194 (10 %ige wässrige Lösung) | 12,00 |
| Wasser, dest. | 30,00 |
| weiterer Zusatz: Konservierungsmittel, lösliches ethoxiliertes Silikon, Parfüm ... | |

| Phase 2: | |
|---|---|
| Natrosol HR 250 (10 %ige Lösung) | 50,00 |
| Hydroxyethylcellulose (Fa. Hercules) | |

### Herstellung:

Die Phasen 1 und 2 werden getrennt eingewogen und homogenisiert. Dann wird Phase 2 langsam in Phase 1 eingerührt. Es bildet sich ein im Wesentlichen klares, stabiles Gel.

Das Beispiel lässt sich mit den Polymeren aus den Beispielen 2, 5, 6 und 7 der EP-A 045 194 wiederholen. Es wird jeweils ein Haargel mit Hydroxyethylcellulose mit guten Eigenschaften erhalten.

### Beispiel 10:

| Conditionershampoo: | | [%] |
|---|---|---|
| A) | Texapon NSO 28 %ig (Natriumlaurethsulfat/Fa. Henkel) | 50,00 |
| | Comperlan KS (Coamid DEA/Fa. Henkel) | 1,00 |
| | Polymer Bsp. 1 EP-A 045 194 (10 %ige wässrige Lösung) | 20,00 |
| | q. s. Parfümöl | |
| B) | Wasser | 27,5 |
| | Natriumchlorid | 1,5 |
| | q. s. Konservierungsmittel ... | |

### Herstellung:

Die Phasen 1 und 2 werden getrennt eingewogen und homogenisiert. Dann wird Phase 2 langsam in Phase 1 eingerührt. Es bildet sich ein im Wesentlichen klares, stabiles Gel.

Das Beispiel lässt sich mit den Polymeren aus den Beispielen 2, 5, 6 und 7 der EP-A 045 194 wiederholen. Es wird jeweils ein Conditionershampoo mit guten Eigenschaften erhalten.

### Beispiel 11:

**Standard O/W-Creme:**

| Ölphase: | [%] | CTFA-Name |
|---|---|---|
| Crempophor A6 | 3,5 | Ceteareth-6 (and) Stearyl Alcohol |
| Crempophor A25 | 3,5 | Ceteareth-25 |
| Glycerinmonostearat s.e. | 2,5 | Glycerylstearat |
| Paraffinöl | 7,5 | Paraffin Oil |
| Cetylalkohol | 2,5 | Cetyl Alcohol |
| Luvitol EHO | 3,2 | Cetearyl Octanoate |
| Vitamin-E-Acetat | 1,0 | Tocopheryl Acetate |
| Nip-Nip | 0,1 | Methyl- and Propyl-4-hydroxybenzoate (7:3) |

**Wasserphase:**

| | | |
|---|---|---|
| Polymer Bsp. 1 EP-A 045 194 (10 %ige wässr. Lösung) | 3,0 | |
| Wasser | 74,6 | |
| 1,2-Propylenglykol | 1,5 | Propylenglykol |
| Germall II | 0,1 | Imidazolidinyl-Harnstoff |

### Herstellung:

Die Öl- und Wasserphasen werden getrennt eingewogen und bei einer Temperatur von ca. 80 °C homogenisiert. Dann wird die Wasserphase langsam in die Ölphase eingerührt und unter Rühren langsam auf Raumtemperatur abgekühlt.

Das Beispiel lässt sich mit den Polymeren aus den Beispielen 2, 5, 6 und 7 der EP-A 045 194 wiederholen. Es wird jeweils eine Standard O/W-Creme mit guten Eigenschaften erhalten.

### Beispiel 12: Flüssiges Makeup

| A | |
|---|---|
| 1,70 | Glycerylstearat |
| 1,70 | Cetylalkohol |
| 1,70 | Ceteareth-6 |
| 1,70 | Ceteareth-25 |
| 5,20 | CapryUCaprin-Triglycerid |
| 5,20 | Mineralöl |

| B | |
|---|---|
| q.s. | Konservierungsmittel |
| 4,30 | Propylenglykol |
| 12,50 | Polymer Bsp. 1 EP-A 045 194 (10 %ige wässrige Lösung) |
| 49,50 | dest. Wasser |

| C | |
|---|---|
| q.s. | Parfumöl |

| D | |
|---|---|
| 2,00 | Eisenoxid |
| 12,00 | Titandioxid |

### Herstellung:

Phase A und Phase B getrennt voneinander auf 80 °C erwärmen. Dann Phase B in Phase A mit einem Rührer mischen. Alles auf 40 °C abkühlen lassen und Phase C und Phase D zugeben. Wiederholt homogenisieren.

Das Beispiel lässt sich mit den Polymeren aus den Beispielen 2, 5, 6 und 7 der EP-A 045 194 wiederholen. Es wird jeweils ein flüssiges Makeup mit guten Eigenschaften erhalten.

### Beispiel 13: Ölfreies Makeup

| A | |
|---|---|
| 0,35 | Veegum |
| 5,00 | Butylenglykol |
| 0,15 | Xanthangummi |

| B | |
|---|---|
| 34,0 | dest. Wasser |
| q.s. | Konservierungsmittel |
| 0,2 | Polysorbate-20 |
| 1,6 | Tetrahydroxypropylethylenediamin |

| C | |
|---|---|
| 1,0 | Siliciumdioxid |
| 2,0 | Nylon-12 |
| 4,15 | Glimmer (mica) |
| 6,0 | Titandioxid |
| 1,85 | Eisenoxid |

| D | |
|---|---|
| 4,0 | Stearinsäure |
| 1,5 | Glycerylstearat |
| 7,0 | Benzyllaurat |
| 5,0 | Isoeicosan |
| q.s. | Konservierungsmittel |

| E | |
|---|---|
| 0,5 | Panthenol |
| 0,1 | Imidazolidinyl-Harnstoff |
| 25,0 | Polymer Bsp. 1 EP-A 045 194 (10 %ige wässrige Lösung) |

### Herstellung:

Phase A mit Butylenglykol benetzen, in Phase B hineingeben und gut mischen. Phase AB auf 75 °C erwärmen. Phase C Einsatzstoffe pulverisieren, in Phase AB hineingeben und gut homogenisieren. Einsatzstoffe von Phase D mischen, auf 80 °C erwärmen und zu Phase ABC geben. Einige Zeit mischen, bis alles homogen ist. Alles in ein Gefäß mit Propellermischer übertragen. Einsatzstoffe von Phase E mischen, in Phase ABCD hineingeben und gut vermischen.

Das Beispiel lässt sich mit den Polymeren aus den Beispielen 2, 5, 6 und 7 der EP-A 045 194 wiederholen. Es wird jeweils ein ölfreies Makeup mit guten Eigenschaften erhalten.

### Beispiel 14: Schimmerndes Gel

| A | |
|---|---|
| 32,6 | dest. Wasser |
| 0,1 | Dinatrium-EDTA |
| 25,0 | Natrosol (4 %ige wässrige Lösung) |
| 0,3 | Konservierungsmittel |

| B | |
|---|---|
| 0,5 | dest. Wasser |
| 0,5 | Triethanolamin |

| C | |
|---|---|
| 9,0 | Polymer Bsp. 1 EP-A 045 194 (10 %ige wässrige Lösung) |
| 1,0 | Polyquaternium-46 (10 %ige wässrige Lösung) |
| 5,0 | Eisenoxid |

| D | |
|---|---|
| 15,0 | dest. Wasser |
| 1,0 | D-Panthenol 50 P (Panthenöl und Propylenglykol) |

### Herstellung:

Mit einem Propellermischer die Einsatzstoffe von Phase A in der angegebenen Reihenfolge gut mischen. Dann Phase B in Phase A hineingeben. Langsam rühren bis alles homogen ist. Phase C gut homogenisieren, bis die Pigmente gut verteilt sind. Phase C und Phase D zu Phase AB geben und gut mischen.

Das Beispiel lässt sich mit den Polymeren aus den Beispielen 2, 5, 6 und 7 der EP-A 045 194 wiederholen. Es wird jeweils ein schimmerndes Gel mit guten Eigenschaften erhalten.

### Beispiel 15: Sonnenschutz-Gel

| Phase A | |
|---|---|
| 1,00 | hydriertes Ricinusöl-PEG-40 |
| 8,00 | Octyl Methoxycinnamate (Uvinul MC 80TM von BASF) |
| 5,00 | Octocrylene (Uvinul N 539 TM von BASF) |
| 0,80 | Octyl Triazone (Uvinul T 150 TM von BASF) |
| 2,00 | Butyl Methoxydibenzoylmethane (Uvinul BMBM TM von BASF) |
| 2,00 | Tocopherylacetat |
| q.s. | Parfümöl |

| Phase B | |
|---|---|
| 12,50 | Polymer Bsp. 1 EP-A 045 194 (10 %ige wässrige Lösung) |
| 0,30 | Acrylat/C₁₀₋₃₀ Alkylacrylat-Copolymer |
| 0,20 | Carbomer |
| 5,00 | Glycerin |
| 0,20 | Dinatrium-EDTA |
| q.s. | Konservierungsmittel |
| 62,80 | dest. Wasser |

| Phase C | |
|---|---|
| 0,20 | Natriumhydroxid |

### Herstellung:

Die Komponenten der Phase A mischen. Phase B quellen lassen und unter Homogenisieren in Phase A einrühren. Mit Phase C neutralisieren und erneut homogenisieren.

Das Beispiel lässt sich mit den Polymeren aus den Beispielen 2, 5, 6 und 7 der EP-A 045 194 wiederholen. Es wird jeweils ein Sonnenschutz-Gel mit guten Eigenschaften erhalten.

### Beispiel 16: Sonnenschutzemulsion mit TiO₂ und ZnO₂

| Phase A | |
|---|---|
| 6,00 | hydriertes Ricinusöl-PEG-7 |
| 2,00 | PEG-45/Dodecyl Glycol Copolymer |
| 3,00 | Isopropylmyristat |
| 8,00 | Jojobaöl (Buxus Chinensis) |
| 4,00 | Octyl Methoxycinnamate (Uvinul MC 80) |
| 2,00 | 4-Methylbenzylidene Camphor (Uvinul MBC 95) |
| 3,00 | Titandioxid, Dimethicon |
| 1,00 | Dimethicon |
| 5,00 | Zinkoxid, Dimethicon |

| Phase B | |
|---|---|
| 10,00 | Polymer Bsp. 1 EP-A 045 194 (10 %ige wässrige Lösung) |
| 0,20 | Dinatrium-EDTA |
| 5,00 | Glycerin |
| q.s. | Konservierungsmittel |
| 50,80 | dest. Wasser |

| Phase C | |
|---|---|
| q.s. | Parfümöl |

### Herstellung:

Die Phasen A und B getrennt auf ca. 85 °C erwärmen. Phase B in Phase A einrühren und homogenisieren. Abkühlen auf ca. 40 °C, Phase C hinzugeben und nochmals kurz homogenisieren.

Das Beispiel lässt sich mit den Polymeren aus den Beispielen 2, 5, 6 und 7 der EP-A 045 194 wiederholen. Es wird jeweils eine Sonnenschutzemulsion mit TiO₂ und ZnO₂ mit guten Eigenschaften erhalten.

### Beispiel 17: Sonnenschutz-Lotion

| Phase A | |
|---|---|
| 6,00 | Octyl Methoxycinnamate (Uvinul MC 80 TM von BASF) |
| 2,50 | 4-Methylbenzylidene Camphor (Uvinul MBC 95 TM von BASF) |
| 1,00 | Octyl Triazone (Uvinul T 150 TM von BASF) |
| 2,00 | Butyl Methoxydibenzoylmethane (Uvinul BMBM TM von BASF) |
| 2,00 | PVP/Hexadecen-Copolymer |
| 5,00 | PPG-3 Myristyl Ether |
| 0,50 | Dimethicon |
| 0,10 | BHT, Ascorbylpalmitat, Citronensäure, Glycerylstearat Propylenglykol |
| 2,00 | Cetylalkohol |
| 2,00 | Kaliumcetylphosphat |

| Phase B | |
|---|---|
| 2,50 | Polymer Bsp. 1 EP-A 045 194 (10 %ige wässrige Lösung) |
| 5,00 | Propylenglykol |
| 0,20 | Dinatrium-EDTA |
| q.s. | Konservierungsmittel |
| 63,92 | dest. Wasser |

| Phase C | |
|---|---|
| 5,00 | Mineralöl |
| 0,20 | Carbomer |

| Phase D | |
|---|---|
| 0,08 | Natriumhydroxid |

| Phase E | |
|---|---|
| q.s. | Parfümöl |

### Herstellung:

Die Phasen A und B getrennt auf ca. 80 °C erwärmen. Phase B unter Homogenisieren in Phase A einrühren, kurz nachhomogenisieren. Phase C anschlämmen, in Phase AB einrühren, mit Phase D neutralisieren und nachhomogenisieren. Abkühlen auf ca. 40°C, Phase E zugeben, nochmals homogenisieren.

Das Beispiel lässt sich mit den Polymeren aus den Beispielen 2, 5, 6 und 7 der EP-A 045 194 wiederholen. Es wird jeweils eine Sonnenschutz-Lotion mit guten Eigenschaften erhalten.

### Beispiel 18: Abziehbare Gesichtsmaske

| Phase A | |
|---|---|
| 57,10 | dest. Wasser |
| 6,00 | Polyvinylalkohol |
| 5,00 | Propylenglykol |

| Phase B | |
|---|---|
| 20,00 | Alkohol |
| 4,00 | PEG-32 |
| q.s | Parfümöl |

| Phase C | |
|---|---|
| 5,00 | Polyquaternium-44 |
| 2,70 | Polymer Bsp. 1 EP-A 045 194 (10 %ige wässrige Lösung) |
| 0,20 | Allantoin |

### Herstellung:

Phase A auf mind. 90 °C erwärmen und rühren bis gelöst. Phase B bei 50 °C lösen und in Phase A einrühren. Bei ca. 35 °C den Ethanolverlust ausgleichen. Phase C zugeben und unterrühren.

Das Beispiel lässt sich mit den Polymeren aus den Beispielen 2, 5, 6 und 7 der EP-A 045 194 wiederholen. Es wird jeweils eine abziehbare Gesichtsmaske mit guten Eigenschaften erhalten.

### Beispiel 19: Gesichtsmaske

| Phase A | |
|---|---|
| 3,00 | Ceteareth-6 |
| 1,50 | Ceteareth-25 |
| 5,00 | Cetearylalkohol |
| 6,00 | Cetearyloctanoat |
| 6,00 | Mineralöl |
| 0,20 | Bisabolol |
| 3,00 | Glycerylstearat |

| Phase B | |
|---|---|
| 2,00 | Propylenglykol |
| 5,00 | Panthenol |
| 14,00 | Polymer Bsp. 1 EP-A 045 194 (10 %ige wässrige Lösung) |
| q.s. | Konservierungsmittel |
| 53,80 | dest. Wasser |

| Phase C | |
|---|---|
| q.s. | Parfümöl |
| 0,50 | Tocopherylacetat |

### Herstellung:

Phase A und B getrennt auf ca. 80 °C erwärmen. Phase B in Phase A unter Homogenisieren einrühren, kurz nachhomogenisieren. Abkühlen auf ca. 40 °C, Phase C hinzugeben, nochmals homogenisieren.

Das Beispiel lässt sich mit den Polymeren aus den Beispielen 2, 5, 6 und 7 der EP-A 045 194 wiederholen. Es wird jeweils eine Gesichtsmaske mit guten Eigenschaften erhalten.

### Beispiel 20: Körperlotion-Schaum

| Phase A | |
|---|---|
| 1,50 | Ceteareth-25 |
| 1,50 | Ceteareth-6 |
| 4,00 | Cetearylalkohol |
| 10,00 | Cetearyloctanoat |
| 1,00 | Dimethicon |

| Phase B | |
|---|---|
| 3,00 | Polymer Bsp. 1 EP-A 045 194 (10 %ige wässrige Lösung) |
| 2,00 | Panthenol |
| 2,50 | Propylenglykol |
| q.s. | Konservierungsmittel |
| 74,50 | dest. Wasser |

| Phase C | |
|---|---|
| q.s. | Parfümöl |

### Herstellung:

Die Phasen A und B getrennt auf ca. 80 °C erwärmen. Phase B in Phase A einrühren und homogenisieren. Abkühlen auf ca. 40 °C, Phase C hinzugeben und nochmals kurz homogenisieren. Abfüllung: 90 % Wirkstoff und 10 % Propan/Butan bei 3,5 bar (20 °C).

Das Beispiel lässt sich mit den Polymeren aus den Beispielen 2, 5, 6 und 7 der EP-A 045 194 wiederholen. Es wird jeweils ein Körperlotion-Schaum mit guten Eigenschaften erhalten.

### Beispiel 21: Gesichtswasser für trockene und empfindliche Haut

| Phase A | |
|---|---|
| 2,50 | hydriertes Rizinusöl-PEG-40 |
| q.s. | Parfümöl |
| 0,40 | Bisabolol |

| Phase B | |
|---|---|
| 3,00 | Glycerin |
| 1,00 | Hydroxyethylcetyldimoniumphosphat |
| 5,00 | Zaubernuss (Hamamelis Virginiana) Destillat |
| 0,50 | Panthenol |
| 0,50 | Polymer Bsp. 1 EP-A 045 194 (10 %ige wässrige Lösung) |
| q.s. | Konservierungsmittel |
| 87,60 | dest. Wasser |

### Herstellung:

Phase A klar lösen. Phase B in Phase A einrühren.

Das Beispiel lässt sich mit den Polymeren aus den Beispielen 2, 5, 6 und 7 der EP-A 045 194 wiederholen. Es wird jeweils ein Gesichtswasser für trockene und empfindliche Haut mit guten Eigenschaften erhalten.

### Beispiel 22: Gesichtswaschpaste mit Peelingeffekt

| Phase A | |
|---|---|
| 58,00 | dest. Wasser |
| 15,00 | Polymer Bsp. 1 EP-A 045 194 (10 %ige wässrige Lösung) |
| 1,50 | Carbomer |
| q.s. | Konservierungsmittel |

| Phase B | |
|---|---|
| q.s. | Parfümöl |
| 7,00 | Potassium Cocoyl Hydrolyzed Protein |
| 4,00 | Cocamidpropylbetain |

| Phase C | |
|---|---|
| 1,50 | Triethanolamin |

| Phase D | |
|---|---|
| 13,00 | Polyethylen (Luwax ATM von BASF) |

### Herstellung:

Phase A quellen lassen. Phase B klar lösen. Phase B in Phase A einrühren. Mit Phase C neutralisieren. Danach Phase D einrühren.

Das Beispiel lässt sich mit den Polymeren aus den Beispielen 2, 5, 6 und 7 der EP-A 045 194 wiederholen. Es wird jeweils eine Gesichtswaschpaste mit Peelingeffekt mit guten Eigenschaften erhalten.

### Beispiel 23: Gesichtsseife

| Phase A | |
|---|---|
| 25,0 | Kaliumcocoat |
| 20,0 | Disodium Cocoamphodiacetate |
| 2.0 | Lauramide DEA |
| 1,0 | Glykolstearat |
| 2,0 | Polymer Bsp. 1 EP-A 045 194 (10 %ige wässrige Lösung) |
| 50,0 | dest. Wasser |
| q.s. | Citronensäure |

| Phase B | |
|---|---|
| q.s. | Konservierungsmittel |
| q.s. | Parfumöl |

### Herstellung:

Phase A unter Rühren auf 70 °C erwärmen, bis alles homogen ist, pH-Wert auf 7,0 - 7,5 mit Zitronensäure einstellen, alles auf 50 °C abkühlen lassen und Phase B zugeben.

Das Beispiel lässt sich mit den Polymeren aus den Beispielen 2, 5, 6 und 7 der EP-A 045 194 wiederholen. Es wird jeweils eine Gesichtsseife mit guten Eigenschaften erhalten.

### Beispiel 24: Gesichtsreinigungsmilch, Typ O/W

| Phase A | |
|---|---|
| 1,50 | Ceteareth-6 |
| 1,50 | Ceteareth-25 |
| 2,00 | Glycerylstearat |
| 2,00 | Cetylalkohol |
| 10,00 | Mineralöl |

| Phase B | |
|---|---|
| 5,00 | Propylenglykol |
| q.s. | Konservierungsmittel |
| 5,0 | Polymer Bsp. 1 EP-A 045 194 (10 %ige wässrige Lösung) |
| 62,30 | dest. Wasser |

| Phase C | |
|---|---|
| 0,20 | Carbomer |
| 10,00 | Cetearyloctanoat |

| Phase D | |
|---|---|
| 0,40 | Tetrahydroxypropylethylendiamin |

| Phase E | |
|---|---|
| q.s. | Parfümöl |
| 0,10 | Bisabolol |

### Herstellung:

Die Phasen A und B getrennt auf ca. 80 °C erwärmen. Phase B unter Homogenisieren in Phase A einrühren, kurz nachhomogenisieren. Phase C anschlämmen, in Phase AB einrühren, mit Phase D neutralisieren und nachhomogenisieren. Abkühlen auf ca. 40 °C, Phase E zugeben, nochmals homogenisieren.

Das Beispiel lässt sich mit den Polymeren aus den Beispielen 2, 5, 6 und 7 der EP-A 045 194 wiederholen. Es wird jeweils eine Gesichtsreinigungsmilch, Typ O/W mit guten Eigenschaften erhalten.

### Beispiel 25: Peeling-Creme, Typ O/W

| Phase A | |
|---|---|
| 3,00 | Ceteareth-6 |
| 1,50 | Ceteareth-25 |
| 3,00 | Glycerylstearat |
| 5,00 | Cetearylalkohol, Sodium Cetearyl Sulfate |
| 6,00 | Cetearyloctanoat |
| 6,00 | Mineralöl |
| 0,20 | Bisabolol |

| Phase B | |
|---|---|
| 2,00 | Propylenglykol |
| 0,10 | Dinatrium-EDTA |
| 3,00 | Polymer Bsp. 1 EP-A 045 194 (10 %ige wässrige Lösung) |
| q.s. | Konservierungsmittel |
| 59,70 | dest. Wasser |

| Phase C | |
|---|---|
| 0,50 | Tocopherylacetat |
| q.s. | Parfümöl |

| Phase D | |
|---|---|
| 10,00 | Polyethylen |

### Herstellung:

Die Phasen A und B getrennt auf ca. 80 °C erwärmen. Phase B in Phase A einrühren und homogenisieren. Abkühlen auf ca. 40 °C, Phase C hinzugeben und nochmals kurz homogenisieren. Anschließend Phase D unterrühren.

Das Beispiel lässt sich mit den Polymeren aus den Beispielen 2, 5, 6 und 7 der EP-A 045 194 wiederholen. Es wird jeweils eine Peeling-Creme, Typ O/W mit guten Eigenschaften erhalten.

### Beispiel 26: Rasierschaum

| | |
|---|---|
| 6,00 | Ceteareth-25 |
| 5,00 | Poloxamer 407 |
| 52,00 | dest. Wasser |
| 1,00 | Triethanolamin |
| 5,00 | Propylenglykol |
| 1,00 | Lanolinöl-PEG-75 |
| 5,00 | erfindungsgemäßes Polymer Bsp. 1 EP-A 045 194 (10 %ige wässrige Lösung) |
| q.s. | Konservierungsmittel |
| q.s. | Parfümöl |
| 25,00 | Sodium Laureth Sulfate |

### Herstellung:

Alles zusammen wiegen, danach rühren bis gelöst. Abfüllung: 90 Teile Wirksubstanz und 10 Teile Propan/Butan-Mischung 25:75.

Das Beispiel lässt sich mit den Polymeren aus den Beispielen 2, 5, 6 und 7 der EP-A 045 194 wiederholen. Es wird jeweils ein Rasierschaum mit guten Eigenschaften erhalten.

### Beispiel 27: After Shave Balsam

| Phase A | |
|---|---|
| 0,25 | Acrylat/C₁₀₋₃₀ Alkylacrylat-Copolymer |
| 1,50 | Tocopherylacetat |
| 0,20 | Bisabolol |
| 10,00 | Capryl/Caprin-Triglycerid |
| q.s. | Parfümöl |
| 1,00 | hydriertes Rizinusöl-PEG-40 |

| Phase B | |
|---|---|
| 1,00 | Panthenol |
| 15,00 | Alkohol |
| 5,00 | Glycerin |
| 0,05 | Hydroxyethylcellulose |
| 1,92 | Polymer Bsp. 1 EP-A 045 194 (10 %ige wässrige Lösung) |
| 64,00 | dest. Wasser |

| Phase C | |
|---|---|
| 0,08 | Natriumhydroxid |

### Herstellung:

Die Komponenten der Phase A mischen. Phase B unter Homogenisieren in Phase A einrühren, kurz nachhomogenisieren. Mit Phase C neutralisieren und erneut homogenisieren.

Das Beispiel lässt sich mit den Polymeren aus den Beispielen 2, 5, 6 und 7 der EP-A 045 194 wiederholen. Es wird jeweils ein After Shave Balsam mit guten Eigenschaften erhalten.

### Beispiel 28: Körperpflegecreme

| Phase A | |
|---|---|
| 2,00 | Ceteareth-6 |
| 2,00 | Ceteareth-25 |
| 2,00 | Cetearylalkohol |
| 3,00 | Glycerylstearat SE |
| 5,00 | Mineralöl |
| 4,00 | Jojobaöl (Buxus Chinensis) |
| 3,00 | Cetearyloctanoat |
| 1,00 | Dimethicon |
| 3,00 | Mineralöl, Lanolinalkohol |

| Phase B | |
|---|---|
| 5,00 | Propylenglykol |
| 0,50 | Veegum |
| 1,00 | Panthenol |
| 8,50 | Polymer Bsp. 1 EP-A 045 194 (10 %ige wässrige Lösung) |
| 6,00 | Polyquaternium-44 (10 %ige wässrige Lösung) |
| q.s. | Konservierungsmittel |
| 54,00 | dest. Wasser |

| Phase C | |
|---|---|
| q.s. | Parfümöl |

### Herstellung:

Die Phasen A und B getrennt auf ca. 80 °C erwärmen. Phase B homogenisieren. Phase B unter Homogenisieren in Phase A einrühren, kurz nachhomogenisieren. Abkühlen auf ca. 40 °C, Phase C hinzugeben und nochmals kurz homogenisieren.

Das Beispiel lässt sich mit den Polymeren aus den Beispielen 2, 5, 6 und 7 der EP-A 045 194 wiederholen. Es wird jeweils eine Körperpflegecreme mit guten Eigenschaften erhalten.

### Beispiel 29: Zahnpasta

| Phase A | |
|---|---|
| 34,79 | dest. Wasser |
| 3,00 | Polymer Bsp. 1 EP-A 045 194 (10 %ige wässrige Lösung) |
| 0,30 | Konservierungsmittel |
| 20,00 | Glycerin |
| 0,76 | Natriummonofluorphosphat |

| Phase B | |
|---|---|
| 1,20 | Natriumcarboxymethylcellulose |

| Phase C | |
|---|---|
| 0,80 | Aromaöl |
| 0,06 | Saccharin |
| 0,10 | Konservierungsmittel |
| 0,05 | Bisabolol |
| 1,00 | Panthenol |
| 0,50 | Tocopherylacetat |
| 2,80 | Siliciumdioxid |
| 1,00 | Natriumlaurylsulfat |
| 7,90 | Dicalciumphosphat, wasserfrei |
| 25,29 | Dicalciumphosphat-Dihydrat |
| 0,45 | Titandioxid |

### Herstellung:

Phase A lösen. Phase B in Phase A einstreuen und lösen. Phase C zugeben und unter Vakuum bei RT ca. 45 Min. rühren lassen.

Das Beispiel lässt sich mit den Polymeren aus den Beispielen 2, 5, 6 und 7 der EP-A 045 194 wiederholen. Es wird jeweils eine Zahnpasta mit guten Eigenschaften erhalten.

### Beispiel 30: Mundwasser

| Phase A | |
|---|---|
| 2,00 | Aromaöl |
| 4,00 | hydriertes Rizinusöl-PEG-40 |
| 1,00 | Bisabolol |
| 30,00 | Alkohol |

| Phase B | |
|---|---|
| 0,20 | Saccharin |
| 5,00 | Glycerin |
| q.s. | Konservierungsmittel |
| 5,00 | Poloxamer 407 |
| 2,5 | Polymer Bsp. 1 EP-A 045 194 (10 %ige wässrige Lösung) |
| 50,30 | dest. Wasser |

### Herstellung:

Phase A und Phase B getrennt klar lösen. Phase B in Phase A einrühren.

Das Beispiel lässt sich mit den Polymeren aus den Beispielen 2, 5, 6 und 7 der EP-A 045 194 wiederholen. Es wird jeweils ein Mundwasser mit guten Eigenschaften erhalten.

### Beispiel 31: Prothesenhaftmittel

| Phase A | |
|---|---|
| 0,20 | Bisabolol |
| 1,00 | Betacarotin |
| q.s. | Aromaöl |
| 20,00 | Cetearyloctanoat |
| 5,00 | Siliciumdioxid |
| 33,80 | Mineralöl |

| Phase B | |
|---|---|
| 5,00 | Polymer Bsp. 1 EP-A 045 194 (10 %ige wässrige Lösung) |
| 35,00 | PVP (10 %ige Lösung in Wasser) |

### Herstellung:

Phase A gut mischen. Phase B in Phase A einrühren.

Das Beispiel lässt sich mit den Polymeren aus den Beispielen 2, 5, 6 und 7 der EP-A 045 194 wiederholen. Es wird jeweils ein Prothesen haftmittel mit guten Eigenschaften erhalten.

### Beispiel 32: Hautpflegecreme, Typ O/W

| Phase A | |
|---|---|
| 8,00 | Cetearylalkohol |
| 2,00 | Ceteareth-6 |
| 2,00 | Ceteareth-25 |
| 10,00 | Mineralöl |
| 5,00 | Cetearyloctanoat |
| 5,00 | Dimethicon |

| Phase B | |
|---|---|
| 3,00 | Polymer Bsp. 1 EP-A 045 194 (10 %ige wässrige Lösung) |
| 2,00 | Panthenol, Propylenglykol |
| q.s. | Konservierungsmittel |
| 63,00 | dest. Wasser |

| Phase C | |
|---|---|
| q.s. | Parfümöl |

### Herstellung:

Phase A und B getrennt auf ca. 80 °C erwärmen. Phase B in Phase A unter Homogenisieren einrühren, kurz nachhomogenisieren. Abkühlen auf ca. 40 °C, Phase C hinzugeben, nochmals homogenisieren.

Das Beispiel lässt sich mit den Polymeren aus den Beispielen 2, 5, 6 und 7 der EP-A 045 194 wiederholen. Es wird jeweils eine Hautpflegecreme, Typ O/W mit guten Eigenschaften erhalten.

### Beispiel 33: Hautpflegecreme, Typ W/O

| Phase A | |
|---|---|
| 6,00 | hydriertes Rizinusöl-PEG-7 |
| 8,00 | Cetearyloctanoat |
| 5,00 | Isopropylmyristat |
| 15,00 | Mineralöl |
| 2,00 | PEG-45/Dodecyl Glycol Copolymer |
| 0,50 | Magnesiumstearat |
| 0,50 | Aluminumstearat |

| Phase B | |
|---|---|
| 3,00 | Glycerin |
| 3,30 | Polymer Bsp. 1 EP-A 045 194 (10 %ige wässrige Lösung) |
| 0,70 | Magnesiumsulfat |
| 2,00 | Panthenol |
| q.s. | Konservierungsmittel |
| 48,00 | dest. Wasser |

| Phase C | |
|---|---|
| 1,00 | Tocopherol |
| 5,00 | Tocopherylacetat |
| q.s. | Parfümöl |

### Herstellung:

Die Phasen A und B getrennt auf ca. 80 °C erwärmen. Phase B in Phase A einrühren und homogenisieren. Abkühlen auf ca. 40 °C, Phase C hinzugeben und nochmals kurz homogenisieren.

Das Beispiel lässt sich mit den Polymeren aus den Beispielen 2, 5, 6 und 7 der EP-A 045 194 wiederholen. Es wird jeweils eine Hautpflegecreme, Typ W/O mit guten Eigenschaften erhalten.

### Beispiel 34: Lippenpflegecreme

| Phase A | |
|---|---|
| 10,00 | Cetearyloctanoat |
| 5,00 | Polybuten |

| Phase B | |
|---|---|
| 0,10 | Carbomer |

| Phase C | |
|---|---|
| 2,00 | Ceteareth-6 |
| 2,00 | Ceteareth-25 |
| 2,00 | Glycerylstearat |
| 2,00 | Cetylalkohol |
| 1,00 | Dimethicon |
| 1,00 | Benzophenone-3 |
| 0,20 | Bisabolol |
| 6,00 | Mineralöl |

| Phase D | |
|---|---|
| 8,00 | Polymer Bsp. 1 EP-A 045 194 (10 %ige wässrige Lösung) |
| 3,00 | Panthenol |
| 3,00 | Propylenglykol |
| q.s. | Konservierungsmittel |
| 54,00 | dest. Wasser |

| Phase E | |
|---|---|
| 0,10 | Triethanolamin |

| Phase F | |
|---|---|
| 0,50 | Tocopherylacetat |
| 0,10 | Tocopherol |
| q.s. | Parfümöl |

### Herstellung:

Phase A klar lösen. Phase B dazugeben und homogenisieren. Phase C addieren und schmelzen bei 80 °C. Phase D auf 80 °C erwärmen. Phase D zu Phase ABC geben und homogenisieren. Abkühlen auf ca. 40 °C, Phase E und Phase F zugeben, nochmals homogenisieren.

Das Beispiel lässt sich mit den Polymeren aus den Beispielen 2, 5, 6 und 7 der EP-A 045 194 wiederholen. Es wird jeweils eine Lippenpflegecreme mit guten Eigenschaften erhalten.

### Beispiel 35: Duschgel

| | |
|---|---|
| 50,00 | Sodium Laureth Sulfate, Magnesium Laureth Sulfate, Sodium Laureth-8 Sulfate, Magnesium Laureth-8 |
| 1,00 | Cocoamide DEA |
| 4,00 | Polymer Bsp. 1 EP-A 045 194 (10 %ige wässrige Lösung) |
| 2,00 | Sodium Laureth Sulfate, Glycol Distearate, Cocamide MEA, Laureth-10 |
| q.s. | Konservierungsmittel |
| q.s. | Parfümöl |
| 2,00 | Natriumchlorid |
| 41,00 | dest. Wasser |

### Herstellung:

Alle Komponenten gemeinsam einwiegen und bis zur Lösung rühren.

Das Beispiel lässt sich mit den Polymeren aus den Beispielen 2, 5, 6 und 7 der EP-A 045 194 wiederholen. Es wird jeweils ein Duschgel mit guten Eigenschaften erhalten.

### Beispiel 36: Duschgel

| | |
|---|---|
| 30,00 | Sodium Laureth Sulfate |
| 6,00 | Sodium Cocoamphodiacetate |
| 6,00 | Cocamidopropylbetain |
| 3,00 | Sodium Laureth Sulfate, Glycol Distearate, Cocamide MEA, Laureth-10 |
| 7,70 | Polyquaternium-44 |
| 1,50 | Polymer Bsp. 1 EP-A 045 194 (10 %ige wässrige Lösung) |
| 1,00 | Panthenol |
| q.s. | Konservierungsmittel |
| q.s. | Parfümöl |
| q.s. | Citronensäure |
| 0,50 | Natriumchlorid |
| 44,30 | dest. Wasser |

### Herstellung:

Die Komponenten der Phase A einwiegen und lösen. Den pH-Wert auf 6 bis 7 einstellen.

Das Beispiel lässt sich mit den Polymeren aus den Beispielen 2, 5, 6 und 7 der EP-A 045 194 wiederholen. Es wird jeweils ein Duschgel mit guten Eigenschaften erhalten.

### Beispiel 37: Klares Duschgel

| | |
|---|---|
| 40,00 | Sodium Laureth Sulfate |
| 5,00 | Decylglukosid |
| 5,00 | Cocamidopropylbetain |
| 0,50 | Polyquaternium-10 |
| 11,00 | Polymer Bsp. 1 EP-A 045 194 (10 %ige wässrige Lösung) |
| 1,00 | Panthenol |
| q.s. | Parfümöl |
| q.s. | Konservierungsmittel |
| q.s. | Citronensäure |
| 2,00 | Natriumchlorid |
| 35,50 | dest. Wasser |

### Herstellung:

Die Komponenten der Phase A einwiegen und klar lösen.

Das Beispiel lässt sich mit den Polymeren aus den Beispielen 2, 5, 6 und 7 der EP-A 045 194 wiederholen. Es wird jeweils ein klares Duschgel mit guten Eigenschaften erhalten.

### Beispiel 38: Duschbad

| A | |
|---|---|
| 40,00 | Sodium Laureth Sulfate |
| 5,00 | Sodium C₁₂₋₁₅ Pareth-15 Sulfonate |
| 5,00 | Decylglukosid |
| q.s. | Parfümöl |
| 0,10 | Phytantriol |

| B | |
|---|---|
| 34,80 | dest. Wasser |
| 0,1 | Guarhydroxypropyltrimoniumchlorid |
| 11,00 | Polymer Bsp. 1 EP-A 045 194 (10 %ige wässrige Lösung) |
| 1,00 | Panthenol |
| q.s. | Konservierungsmittel |
| 1,00 | Laureth-3 |
| q.s. | Citronensäure |
| 2,00 | Natriumchlorid |

### Herstellung:

Die Komponenten der Phase A mischen. Die Komponenten der Phase B nacheinander zugeben und mischen. Den pH-Wert auf 6 bis 7 einstellen.

Das Beispiel lässt sich mit den Polymeren aus den Beispielen 2, 5, 6 und 7 der EP-A 045 194 wiederholen. Es wird jeweils ein Duschbad mit guten Eigenschaften erhalten.

### Beispiel 39: Flüssigseife

| A | |
|---|---|
| 43,26 | dest. Wasser |
| 0,34 | Aminomethylpropanol |
| 3,40 | Poly(Ethylacrylat/Methacrylsäure) (Luviflex Soft®, Fa. BASF) |

| B | |
|---|---|
| 40,00 | Sodium Laureth Sulfate |
| 10,00 | Cocamidopropylbetain |
| 1,00 | Polymer Bsp. 1 EP-A 045 194 (10 %ige wässrige Lösung) |
| q.s. | Parfümöl |
| q.s. | Konservierungsmittel |
| 2,00 | Natriumchlorid |

### Herstellung:

Die Komponenten der Phase A einwiegen und klar lösen. Die Komponenten der Phase B nacheinander zugeben und mischen.

Das Beispiel lässt sich mit den Polymeren aus den Beispielen 2, 5, 6 und 7 der EP-A 045 194 wiederholen. Es wird jeweils eine Flüssigseife mit guten Eigenschaften erhalten.

### Beispiel 40: Flüssiges Fußbad

| A | |
|---|---|
| 1,00 | Nonoxynol-14 |
| 0,10 | Bisabolol |
| 1,00 | Pinienöl (Pinus Sylvestris) |

| B | |
|---|---|
| 5,00 | PEG-8 |
| 6,00 | Polymer Bsp. 1 EP-A 045 194 (10 %ige wässrige Lösung) |
| 0,50 | Triclosan |
| 30,00 | Sodium Laureth Sulfate |
| 3,00 | Polyquaternium-16 |
| 53,40 | dest. Wasser |
| q.s. | C. I. 19 140 + C. I. 42 051 |

### Herstellung:

Phase A solubilisieren. Phase B mischen.

Das Beispiel lässt sich mit den Polymeren aus den Beispielen 2, 5, 6 und 7 der EP-A 045 194 wiederholen. Es wird jeweils ein flüssiges Fußbad mit guten Eigenschaften erhalten.

### Beispiel 41: Erfrischungsgel

| A | |
|---|---|
| 0,60 | Carbomer |
| 45,40 | dest. Wasser |

| B | |
|---|---|
| 0,50 | Bisabolol |
| 0,50 | Farnesol |
| q.s. | Parfümöl |
| 5,00 | PEG-40 Hydrogenated Castor Oil |
| 2,50 | Polymer Bsp. 1 EP-A 045 194 (10 %ige wässrige Lösung) |
| 1,00 | Tetrahydroxypropylethylendiamin |
| 1,50 | Menthol |
| 43,00 | Alkohol |
| q.s. | C.l. 74 180, Direct Blue 86 |

### Herstellung:

Phase A quellen lassen. Phase B lösen. Phase B in Phase A einrühren.

Das Beispiel lässt sich mit den Polymeren aus den Beispielen 2, 5, 6 und 7 der EP-A 045 194 wiederholen. Es wird jeweils ein Erfrischungsgel mit guten Eigenschaften erhalten.

### Beispiel 42: Roll-on Antiperspirant

| A | |
|---|---|
| 0,40 | Hydroxyethylcellulose |
| 50,00 | dest. Wasser |

| B | |
|---|---|
| 25,00 | Alkohol |
| 0,10 | Bisabolol |
| 0,30 | Farnesol |
| 2,00 | PEG-40 Hydrogenated Castor Oil |
| q.s. | Parfümöl |

| C | |
|---|---|
| 5,00 | Aluminumchlorhydrat |
| 3,00 | Propylenglykol |
| 3,00 | Dimethicon-Copolyol |
| 3,00 | Polyquaternium-16 |
| 6,00 | Polymer Bsp. 1 EP-A 045 194 (10 %ige wässrige Lösung) |
| 2,20 | dest. Wasser |

### Herstellung:

Phase A quellen lassen. Phase B und C getrennt lösen. Phase A und B in Phase C einrühren.

Das Beispiel lässt sich mit den Polymeren aus den Beispielen 2, 5, 6 und 7 der EP-A 045 194 wiederholen. Es wird jeweils ein Roll-on Antiperspirant mit guten Eigenschaften erhalten.

### Beispiel 43: Transparenter Deostift

| | |
|---|---|
| 5,00 | Natriumstearat |
| 0,50 | Triclosan |
| 3,00 | Ceteareth-25 |
| 20,00 | Glycerin |
| 2,50 | Polymer Bsp. 1 EP-A 045 194 (10 %ige wässrige Lösung) |
| q.s. | Parfümöl |
| 60,00 | Propylenglykol |
| 0,20 | Bisabolol |
| 10,80 | dest. Wasser |

### Herstellung:

Phase A zusammenwiegen, schmelzen und homogenisieren. Anschließend in die Form gießen.

Das Beispiel lässt sich mit den Polymeren aus den Beispielen 2, 5, 6 und 7 der EP-A 045 194 wiederholen. Es wird jeweils ein transparenter Deostift mit guten Eigenschaften erhalten.

### Beispiel 44: Wasserlösliches Badeöl

| | |
|---|---|
| 15,00 | Cetearyloctanoat |
| 15,00 | Capryl/Caprin-Triglycerid |
| 1,00 | Panthenol, Propylenglykol |
| 0,10 | Bisabolol |
| 2,00 | Tocopherylacetat |
| 2,00 | Retinylpalmitat |
| 0,10 | Tocopherol |
| 37,00 | PEG-7-Glyceryl-Cocoate |
| 2,00 | Polymer Bsp. 1 EP-A 045 194 (10 %ige wässrige Lösung) |
| 2,20 | dest. Wasser |
| q.s. | Parfümöl |
| 23,60 | PEG-40 Hydrogenated Castor Oil |

### Herstellung:

Mischen und rühren bis alles klar gelöst ist.

Das Beispiel lässt sich mit den Polymeren aus den Beispielen 2, 5, 6 und 7 der EP-A 045 194 wiederholen. Es wird jeweils ein wasserlösliches Badeöl mit guten Eigenschaften erhalten.

### Beispiel 45: Tagespflege-Aerosol

| A | |
|---|---|
| 4,00 | Ethylhexyl Methoxycinnamate |
| 1,50 | Octocrylen |
| 9,00 | Capryl/Caprin-Triglycerid |
| 5,00 | Simmondsia Chinensis (Jojoba) Seed Oil |
| 1,50 | Cyclomethicon |
| 3,00 | Hydrogenated Coco-Glycerides |
| 1,00 | PVP/Hexadecen-Copolymer |
| 1,00 | Ceteareth-6, Stearylalkohol |

| B | |
|---|---|
| 5,00 | Zinkoxid |

| C | |
|---|---|
| 2,00 | Ceteareth-25 |
| 1,20 | Panthenol |
| 0,20 | Sodium Ascorbyl Phosphate |
| 0,30 | Imidazolidinyl-Harnstoff |
| 0,10 | Disodium EDTA |
| 7,50 | Polymer Bsp. 1 EP-A 045 194 (10 %ige wässrige Lösung) |
| 56,67 | dest. Wasser |

| D | |
|---|---|
| 0,50 | Tocopherylacetat |
| 0,20 | Bisabolol |
| 0,33 | Capryl/Caprin-Triglycerid, Retinol |
| q.s. | Parfümöl |

### Herstellung:

Phase A auf 80 °C erwärmen. Phase A klar lösen. Phase B einarbeiten und homogenisieren. Phase C zugeben, erhitzen auf 80 °C, aufschmelzen und homogenisieren. Unter Rühren auf ca. 40 °C abkühlen, Phase D hinzugeben und kurz homogenisieren. 90 % Wirkstofflösung: 10 % Propan/Butan mit 3,5 bar (20 °C) abfüllen.

Das Beispiel lässt sich mit den Polymeren aus den Beispielen 2, 5, 6 und 7 der EP-A 045 194 wiederholen. Es wird jeweils ein Tagespflege-Aerosol mit guten Eigenschaften erhalten.

### Beispiel 46: Feuchtigkeitscreme

| A | |
|---|---|
| 3,00 | Vitis Vinifera (Grape) Seed Oil |
| 1,00 | Cyclopentasiloxan, Cyclohexasiloxan |
| 1,50 | Cyclomethicon |
| 2,00 | Soybean (Glycine Soja) Oil |
| 2,00 | Ethylhexyl Methoxycinnamate |
| 1,00 | Uvinul A Plus (BASF) |
| 1,00 | Hydrogenated Lecithin |
| 1,00 | Cholesterol |
| 2,00 | PEG-40 Hydrogenated Castor Oil |
| 5,00 | Cetearyloctanoat |
| 5,00 | Capryl/Caprin-Triglycerid |

| B | |
|---|---|
| 3,00 | Capry/Caprin-Triglycerid, Acrylat-Copolymer |

| C | |
|---|---|
| 3,00 | Polymer Bsp. 1 EP-A 045 194 (10 %ige wässrige Lösung) |
| 0,50 | Cocotrimoniummethsulfat |
| 2,00 | Panthenol, Propylenglykol |
| 3,00 | Glycerin |
| 0,10 | Dinatrium-EDTA |
| 60,30 | dest. Wasser |

| D | |
|---|---|
| 0,30 | Parfümöl |
| 0,30 | DMDM Hydantoin |
| 1,00 | Tocopherylacetat |
| 2,00 | Tocopherol |

### Herstellung:

Phase A auf 80 °C erwärmen. Phase B in Phase A einrühren. Phase C auf ca. 80 °C erwärmen und unter Homogenisieren in Phase A+B einrühren. Unter Rühren auf ca. 40 °C abkühlen, Phase D hinzugeben und kurz homogenisieren.

Das Beispiel lässt sich mit den Polymeren aus den Beispielen 2, 5, 6 und 7 der EP-A 045 194 wiederholen. Es wird jeweils eine Feuchtigkeitscreme mit guten Eigenschaften erhalten.

### Beispiel 47: Aerosol-Haarschaum

| A | |
|---|---|
| 2,00 | Cocotrimoniummethsulfat |
| 0,20 | Parfümöl |

| B | |
|---|---|
| 63,90 | dest. Wasser |
| 6,70 | Polymer Bsp. 1 EP-A 045 194 (10 %ige wässrige Lösung) |
| 0,50 | Poly(Ethylacrylat/Methacrylsäure) (Luviflex Soft®, Fa. BASF) |
| 0,10 | Aminomethylpropanol |
| 0,20 | Ceteareth-25 |
| 0,20 | Trimethylsilylamodimethicon, Trideceth-10, Cetrimonium Chloride |
| 0,10 | PEG-25 PABA |
| 0,20 | Hydroxyethylcellulose |
| 0,20 | PEG-8 |
| 0,20 | Panthenol |
| 15,00 | Alkohol |

| C | |
|---|---|
| 10,00 | Propan/Butan 3,5 bar (20 °C) |

### Herstellung:

Phasen A und B mischen und mit Treibgas abfüllen.

Das Beispiel lässt sich mit den Polymeren aus den Beispielen 2, 5, 6 und 7 der EP-A 045 194 wiederholen. Es wird jeweils ein Aerosol-Haarschaum mit guten Eigenschaften erhalten.

### Beispiel 48: Pumpmousse

| A | |
|---|---|
| 2,00 | Cocotrimoniummethosulfat |
| q.s. | Parfümöl |

| C | |
|---|---|
| 74,30 | dest. Wasser |
| 7,00 | Polyquaternium-46 (10 %ige wässrige Lösung) |
| 15,00 | Polymer Bsp. 1 EP-A 045 194 (10 %ige wässrige Lösung) |
| 0,50 | PEG-8 |
| 1,00 | Panthenol |
| q.s. | Konservierungsmittel |
| 0,20 | PEG-25 PABA (ethoxilierte p-Aminobenzoesäure) |

### Herstellung:

Die Komponenten der Phase A mischen. Die Komponenten der Phase B nacheinander zugeben und klar lösen.

Das Beispiel lässt sich mit den Polymeren aus den Beispielen 2, 5, 6 und 7 der EP-A 045 194 wiederholen. Es wird jeweils ein Pumpmousse mit guten Eigenschaften erhalten.

### Beispiel 49: Aerosolschaum

| | |
|---|---|
| 15,00 | Polymer Bsp. 1 EP-A 045 194 (10 %ige wässrige Lösung) |
| 5,00 | PVP/VA-Copolymer (40 %ige wässrige Lösung) |
| 0,50 | Hydroxyethylcetyldimoniumphosphat |
| 0,20 | Ceteareth-25 |
| 0,40 | Parfümöl PC 910.781/Cremophor |
| 68,90 | dest. Wasser |
| q.s. | Konservierungsmittel |
| 10,00 | Propan/Butan 3,5 bar (20 °C) |

### Herstellung:

Alles zusammen wiegen, rühren bis gelöst, dann abfüllen.

Das Beispiel lässt sich mit den Polymeren aus den Beispielen 2, 5, 6 und 7 der EP-A 045 194 wiederholen. Es wird jeweils ein Aerosolschaum mit guten Eigenschaften erhalten.

### Beispiel 50: Farbstyling-Mousse

| A | |
|---|---|
| 2,00 | Cocotrimoniummethosulfat |
| q.s. | Parfümöl |

| B | |
|---|---|
| 33,50 | Polymer Bsp. 1 EP-A 045 194 (10 %ige wässrige Lösung) |
| 0,50 | Acrylatcopolymer (Luvimer 100 P®, Fa. BASF) |
| 0,10 | Aminomethylpropanol |
| 0,20 | Ceteareth-25 |
| 0,20 | Panthenol |
| 0,20 | Hydroxyethylcellulose |
| 10,00 | Alkohol |
| 43,17 | dest. Wasser |
| 0,08 | C.I. 12245, Basic Red 76 |
| 0,05 | C.I. 42510, Basic Violet 14 |

| C | |
|---|---|
| 10,00 | Propan/Butan 3,5 bar (20 °C) |

### Herstellung:

Alles zusammen wiegen, rühren bis gelöst, dann abfüllen. Nur für dunkelblondes und braunes Haar geeignet!

Das Beispiel lässt sich mit den Polymeren aus den Beispielen 2, 5, 6 und 7 der EP-A 045 194 wiederholen. Es wird jeweils ein Farbstyling-Mousse mit guten Eigenschaften erhalten.

### Beispiel 51: Pumphaarschaum

| A | |
|---|---|
| 1,50 | Cocotrimoniummethosulfat |
| q.s. | Parfümöl |

| B | |
|---|---|
| 10,00 | Polymer Bsp. 1 EP-A 045 194 (10 %ige wässrige Lösung) |
| 86,04 | dest. Wasser |

| C | |
|---|---|
| 0,46 | Aminomethylpropanol |
| 4,00 | PEG/PPG-25/25 Dimethicon/Acrylat-Copolymer |
| q.s. | Konservierungsmittel |

### Herstellung:

Phase A mischen. Phase B in Phase A einrühren. Phase C zugeben und rühren bis gelöst.

Das Beispiel lässt sich mit den Polymeren aus den Beispielen 2, 5, 6 und 7 der EP-A 045 194 wiederholen. Es wird jeweils ein Pumphaarschaum mit guten Eigenschaften erhalten.

### Beispiel 52: Aquawax

| | |
|---|---|
| 50,00 | Polymer Bsp. 1 EP-A 045 194 (10 %ige wässrige Lösung) |
| q.s. | Parfümöl |
| q.s. | hydriertes Rizinusöl-PEG-40 |
| 0,10 | Diethylphthalat |
| 0,10 | Cetearylethylhexanoat |
| 0,10 | PEG-7 Glyceryl Cocoate |
| 0,10 | Konservierungsmittel |
| 47,70 | dest. Wasser |
| 2,00 | Capryl/Caprin-Triglycerid, Acrylat-Copolymer |

### Herstellung:

Alles mischen und homogenisieren. 15 Minuten nachrühren.

Das Beispiel lässt sich mit den Polymeren aus den Beispielen 2, 5, 6 und 7 der EP-A 045 194 wiederholen. Es wird jeweils ein Aquawax mit guten Eigenschaften erhalten.

### Beispiel 53: Rinse-off Conditioner und Repair Treatment

| A | |
|---|---|
| 0,20 | Cetearyloctanoat |
| 0,10 | Phytantriol |
| 2,00 | hydriertes Rizinusöl-PEG-40 |

| B | |
|---|---|
| q.s. | Parfümöl |
| 2,00 | Cocotrimoniummethosulfat |

| C | |
|---|---|
| 77,70 | dest. Wasser |

| D | |
|---|---|
| 2,00 | Polyquaternium-16 (10 %ige wässrige Lösung) |
| 5,00 | Polymer Bsp. 1 EP-A 045 194 (10 %ige wässrige Lösung) |
| 1,00 | Dimethicon-Copolyol |
| q.s. | Konservierungsmittel |
| 10,00 | Alkohol |
| q.s. | Citronensäure |

### Herstellung:

Die Phasen A und B getrennt mischen. Phase C in Phase B einrühren.

Das Beispiel lässt sich mit den Polymeren aus den Beispielen 2, 5, 6 und 7 der EP-A 045 194 wiederholen. Es wird jeweils ein Rinse-off Conditioner und Repair Treatment mit guten Eigenschaften erhalten.

### Beispiel 54: Haarkur

| A | |
|---|---|
| 2,00 | Ceteareth-6, Stearylalkohol |
| 1,00 | Ceteareth-25 |
| 6,00 | Cetearylalkohol |
| 6,00 | Cetearyloctanoat |
| 0,30 | Phytantriol |

| B | |
|---|---|
| 5,00 | Polymer Bsp. 1 EP-A 045 194 (10 %ige wässrige Lösung) |
| 0,70 | Guar Hydroxypropytrimonoium Chloride |
| 5,00 | Propylenglykol |
| 2,00 | Panthenol |
| 0,30 | Imidazolidinyl-Harnstoff |
| 69,00 | dest. Wasser |

| C | |
|---|---|
| 2,00 | Cosi Silk Soluble |
| 0,20 | Parfum |
| 0,50 | Phenoxyethanol |

### Herstellung:

Die Phasen A und B getrennt auf ca. 80 °C erwärmen. Phase B homogenisieren.

Das Beispiel lässt sich mit den Polymeren aus den Beispielen 2, 5, 6 und 7 der EP-A 045 194 wiederholen. Es wird jeweils eine Haarkur mit guten Eigenschaften erhalten.

### Beispiel 55: Haar-Cocktail

| A | |
|---|---|
| 0,40 | Acrylate/C₁₀₋₃₀ Alkylacrylat-Crosspolymer |
| 2,00 | Dimethicon |
| 3,00 | Cyclomethicon, Dimethiconol |
| 2,00 | Phenyltrimethicon |
| 2,00 | Amodimethicone, Cetrimonium Chloride, Trideceth-10 |
| 0,50 | Dimethicon-Copolyol |
| 1,00 | Macadamia-Nussöl (Ternifolia) |
| 0,50 | Tocopherylacetat |
| 1,00 | PEG-40 Hydrogenated Castor Oil |
| q.s. | Parfümöl |

| B | |
|---|---|
| 81,64 | dest. Wasser |
| 1,50 | Polymer Bsp. 1 EP-A 045 194 (10 %ige wässrige Lösung) |
| 0,46 | Aminomethylpropanol |
| 4,00 | PEG/PPG-25/25 Dimethicon/Acrylat-Copolymer |

### Herstellung:

Die Komponenten der Phase A mischen. Phase B lösen. Phase B unter Homogenisieren in Phase A einrühren.

Das Beispiel lässt sich mit den Polymeren aus den Beispielen 2, 5, 6 und 7 der EP-A 045 194 wiederholen. Es wird jeweils ein Haar-Cocktail mit guten Eigenschaften erhalten.

### Beispiel 56: Dauerwelle

Well-Lösung

| A | |
|---|---|
| 68,95 | dest. Wasser |
| 0,20 | Cocamidopropylbetain |
| 0,20 | Polysorbate 20 |
| 6,25 | erfindungsgemäßes Polymer Bsp. 1 EP-A 045 194 (10 %ige wässrige Lösung) |
| 0,20 | Dinatrium-EDTA |
| 0,20 | Hydroxyethylcellulose |

| B | |
|---|---|
| 8,00 | Thioglykolsäure |

| C | |
|---|---|
| 11,00 | Ammoniumhydroxid |

| D | |
|---|---|
| 5,00 | Ammoniumcarbonat |

### Herstellung:

Die Komponenten der Phase A einwiegen und klar lösen. Phase B in Phase A einrühren.

Das Beispiel lässt sich mit den Polymeren aus den Beispielen 2, 5, 6 und 7 der EP-A 045 194 wiederholen. Es wird jeweils eine Well-Lösung mit guten Eigenschaften erhalten.

### Beispiel 57: Fixierung:

| A | |
|---|---|
| 1,00 | PEG-40 Hydrogenated Castor Oil |
| 0,20 | Parfümöl |
| 83,60 | dest. Wasser |

| B | |
|---|---|
| 0,20 | Cocamidopropylbetain |
| 0,20 | Ceteareth-25 |
| 12,50 | Polymer Bsp. 1 EP-A 045 194 (10 %ige wässrige Lösung) |
| q.s. | Konservierungsmittel |

| C | |
|---|---|
| 2,30 | Wasserstoffperoxid |

| D | |
|---|---|
| q.s. | Phosphorsäure |

### Herstellung:

Phase A solubilisieren. Die Komponenten der Phase B nacheinander zugeben und klar lösen.

Das Beispiel lässt sich mit den Polymeren aus den Beispielen 2, 5, 6 und 7 der EP-A 045 194 wiederholen. Es wird jeweils eine Fixierung mit guten Eigenschaften erhalten.

### Beispiel 58: dunkelbraune Permanenthaarfarbe (Oxidationshaarfarbe)

| A | |
|---|---|
| 46,90 | dest. Wasser |
| 0,20 | Natriumsulfit |
| 0,05 | Dinatrium-EDTA |
| 0,20 | p-Phenylendiamin |
| 0,30 | Resorcinol |
| 0,20 | 4-Amino-2-hydroxytoluol |
| 0,10 | m-Aminophenol |
| 1,50 | Oleylalkohol |
| 4,50 | Propylenglykol |
| 2,30 | Sodium C₁₂₋₁₅ Pareth-15 Sulfonate |
| 20,00 | Ölsäure |

| B | |
|---|---|
| 5,00 | Polymer Bsp. 1 EP-A 045 194 (10 %ige wässrige Lösung) |
| 13,70 | Ammoniumhydroxid |
| 6,00 | i-Propanol |
| q.s. | Parfum |

### Herstellung:

Phase A solubilisieren. Die Komponenten der Phase B nacheinander zugeben und mischen.

Das Beispiel lässt sich mit den Polymeren aus den Beispielen 2, 5, 6 und 7 der EP-A 045 194 wiederholen. Es wird jeweils eine dunkelbraune Permanenthaarfarbe (Oxidationshaarfarbe) mit guten Eigenschaften erhalten.

### Beispiel 59: Entwickleremulsion (pH 3 -4)

| | |
|---|---|
| 3,00 | Hexadecylalkohol |
| 10,00 | Polymer Bsp. 1 EP-A 045 194 (10 %ige wässrige Lösung) |
| 1,00 | Ceteareth-20 |
| 1,00 | Sodium C₁₂₋₁₅ Pareth-15 Sulfonate |
| 6,00 | Wasserstoffperoxid |
| 0,50 | Phosphorsäure |
| 0,01 | Acetanilid |
| 78,49 | dest. Wasser |

### Herstellung:

Die Komponenten nacheinander zugeben und mischen.

Das Beispiel lässt sich mit den Polymeren aus den Beispielen 2, 5, 6 und 7 der EP-A 045 194 wiederholen. Es wird jeweils eine Entwickleremulsion (pH 3 - 4) mit guten Eigenschaften erhalten.

### Beispiel 60: hellbraune Semi-Permanenthaarfarbe

| | |
|---|---|
| 10,00 | Cocodiethanolamid |
| 4,00 | Natriumdodecylbenzylsulfonat, 50 %ig |
| 5,00 | Polymer Bsp. 1 EP-A 045 194 (10 %ige wässrige Lösung) |
| 6,00 | C₉₋₁₁ Pareth-3 |
| 2,50 | Natriumlaurylsulfat |
| 0,40 | 2-Nitro-p-phenylendiamin |
| 0,20 | HC Red No.3 |
| 0,20 | HC Yellow No.2 |
| 71,70 | dest. Wasser |

### Herstellung:

Die Komponenten nacheinander zugeben und mischen.

Das Beispiel lässt sich mit den Polymeren aus den Beispielen 2, 5, 6 und 7 der EP-A 045 194 wiederholen. Es wird jeweils eine hellbraune Semi-Permanenthaarfarbe mit guten Eigenschaften erhalten.

### Beispiel 61: Shampoo

| | |
|---|---|
| 30,00 | Sodium Laureth Sulfate |
| 6,00 | Sodium Cocoamphoacetate |
| 6,00 | Cocamidopropylbetain |
| 3,00 | Sodium Laureth Sulfate, Glycol Distearate, Cocamide MEA, Laureth-10 |
| 5,00 | Polymer Bsp. 1 EP-A 045 194 (10 %ige wässrige Lösung) |
| 2,00 | Dimethicon |
| q.s. | Parfum |
| q.s. | Konservierungsstoff |
| q.s. | Citronensäure |
| 1,00 | Natriumchlorid |
| ad 100 | dest. Wasser |

### Herstellung:

Komponenten einwiegen und lösen. pH-Wert auf 6 bis 7 einstellen.

Das Beispiel lässt sich mit den Polymeren aus den Beispielen 2, 5, 6 und 7 der EP-A 045 194 wiederholen. Es wird jeweils ein Shampoo mit guten Eigenschaften erhalten.

### Beispiel 62: Shampoo

| | |
|---|---|
| 30,00 | Sodium Laureth Sulfate |
| 6,00 | Sodium Cocoamphoacetate |
| 6,00 | Cocamidopropylbetain |
| 3,00 | Sodium Laureth Sulfate, Glykol Distearate, Cocamide MEA, Laureth-10 |
| 5,00 | Polymer Bsp. 1 EP-A 045 194 (10 %ige wässrige Lösung) |
| 2,00 | Amodimethicon |
| q.s. | Parfüm |
| q.s. | Konservierungsstoff |
| q.s. | Citronensäure |
| 1,00 | Natriumchlorid |
| ad 100 | dest. Wasser |

### Herstellung:

Komponenten einwiegen und lösen. pH-Wert auf 6 bis 7 einstellen.

Das Beispiel lässt sich mit den Polymeren aus den Beispielen 2, 5, 6 und 7 der EP-A 045 194 wiederholen. Es wird jeweils ein Shampoo mit guten Eigenschaften erhalten.

### Beispiel 63: Shampoo

| | |
|---|---|
| 40,00 | Sodium Laureth Sulfate |
| 10,00 | Cocamidopropylbetain |
| 3,00 | Sodium Laureth Sulfate, Glykol Distearate, Cocamide MEA, Laureth-10 |
| 5,00 | Polymer Bsp. 1 EP-A 045 194 (10 %ige wässrige Lösung) |
| 2,00 | Dow Corning 3052 |
| q.s. | Parfum |
| q.s. | Konservierungsstoff |
| q.s. | Citronensäure |
| 2,00 | Cocamido DEA |
| ad 100 | dest. Wasser |

### Herstellung:

Komponenten einwiegen und lösen. pH-Wert auf 6 bis 7 einstellen.

Das Beispiel lässt sich mit den Polymeren aus den Beispielen 2, 5, 6 und 7 der EP-A 045 194 wiederholen. Es wird jeweils ein Shampoo mit guten Eigenschaften erhalten.

### Beispiel 64: Antischuppen-Shampoo

| | |
|---|---|
| 40,00 | Sodium Laureth Sulfate |
| 10,00 | Cocamidopropylbetain |
| 10,00 | Disodium Laureth Sulfosuccinate |
| 2,50 | Sodium Laureth Sulfate, Glycol Distearate, Cocamide MEA, Laureth-10 |
| 5,00 | Polymer Bsp. 1 EP-A 045 194 (10 %ige wässrige Lösung) |
| 0,50 | Climbazol |
| q.s. | Parfum |
| q.s. | Konservierungsstoff |
| 0,50 | Natriumchlorid |
| ad 100 | dest. Wasser |

### Herstellung:

Komponenten einwiegen und lösen. pH-Wert auf 6 bis 7 einstellen.

Das Beispiel lässt sich mit den Polymeren aus den Beispielen 2, 5, 6 und 7 der EP-A 045 194 wiederholen. Es wird jeweils ein Antischuppen-Shampoo mit guten Eigenschaften erhalten.

### Beispiel 65: Shampoo

| | |
|---|---|
| 25,00 | Sodium Laureth Sulfate |
| 5,00 | Cocamidopropylbetain |
| 2,50 | Sodium Laureth Sulfate, Glycol Distearate,Cocamide MEA, Laureth-10 |
| 5,00 | Polymer Bsp. 1 EP-A 045 194 (10 %ige wässrige Lösung) |
| q.s. | Parfum |
| q.s. | Konservierungsstoff |
| 2,00 | Cocamido DEA |
| ad 100 | dest. Wasser |

### Herstellung:

Komponenten einwiegen und lösen. pH-Wert auf 6 bis 7 einstellen.

Das Beispiel lässt sich mit den Polymeren aus den Beispielen 2, 5, 6 und 7 der EP-A 045 194 wiederholen. Es wird jeweils ein Shampoo mit guten Eigenschaften erhalten.

### Beispiel 66: Shampoo

| | |
|---|---|
| 20,00 | Ammonium Laureth Sulfate |
| 15,00 | Ammonium Lauryl Sulfate |
| 5,00 | Cocamidopropylbetain |
| 2,50 | Sodium Laureth Sulfate, Glycol Distearate,Cocamide MEA, Laureth-10 |
| 5,00 | Polymer Bsp. 1 EP-A 045 194 (10 %ige wässrige Lösung) |
| q.s. | Parfum |
| q.s. | Konservierungsstoff |
| 0,50 | Natriumchlorid |
| ad 100 | dest. Wasser |

### Herstellung:

Komponenten einwiegen und lösen. pH-Wert auf 6 bis 7 einstellen.

Das Beispiel lässt sich mit den Polymeren aus den Beispielen 2, 5, 6 und 7 der EP-A 045 194 wiederholen. Es wird jeweils ein Shampoo mit guten Eigenschaften erhalten.

### Beispiel 67: Klares Duschgel

| | |
|---|---|
| 40,00 | Sodium Laureth Sulfate |
| 5,00 | Decylglukosid |
| 5,00 | Cocamidopropylbetain |
| 5,00 | Polymer Bsp. 1 EP-A 045 194 (10 %ige wässrige Lösung) |
| 1,00 | Panthenol |
| q.s. | Parfum |
| q.s. | Konservierungsstoff |
| q.s. | Citronensäure |
| 2,00 | Natriumchlorid |
| ad 100 | dest. Wasser |

### Herstellung:

Komponenten einwiegen und lösen. pH-Wert auf 6 bis 7 einstellen.

Das Beispiel lässt sich mit den Polymeren aus den Beispielen 2, 5, 6 und 7 der EP-A 045 194 wiederholen. Es wird jeweils ein klares Duschgel mit guten Eigenschaften erhalten.

### Beispiel 68: Shampoo

| | |
|---|---|
| 12,00 | Sodium Laureth Sulfate |
| 1,50 | Decylglukosid |
| 2,50 | Cocamidopropylbetain |
| 5,00 | Coco-Glucoside Glyceryl Oleate |
| 2,00 | Sodium Laureth Sulfate, Glycol Distearate,Cocamide MEA,Laureth-10 |
| 5,00 | Polymer Bsp. 1 EP-A 045 194 (10 %ige wässrige Lösung) |
| q.s. | Konservierungsstoff |
| q.s. | Sunset Yellow C. I. 15 985 |
| q.s. | Parfum |
| 1,00 | Natriumchlorid |
| ad 100 | dest. Wasser |

### Herstellung:

Komponenten einwiegen und lösen. pH-Wert auf 6 bis 7 einstellen.

Das Beispiel lässt sich mit den Polymeren aus den Beispielen 2, 5, 6 und 7 der EP-A 045 194 wiederholen. Es wird jeweils ein Shampoo mit guten Eigenschaften erhalten.

### Beispiel 69: Shampoo

| A | |
|---|---|
| 40,00 | Sodium Laureth Sulfate |
| 5,00 | SodiumC₁₂₋₁₅Pareth-15 Sulfonate |
| 5,00 | Decylglukosid |
| q.s. | Parfum |
| 0,10 | Phytantriol |

| B | |
|---|---|
| ad 100 | dest. Wasser |
| 5,00 | Polymer Bsp. 1 EP-A 045 194 (10 %ige wässrige Lösung) |
| 1,00 | Panthenol |
| q.s. | Konservierungsstoff |
| 1,00 | Laureth-3 |
| q.s. | Citronensäure |
| 2,00 | Natriumchlorid |

### Herstellung:

Komponenten der Phase A einwiegen und lösen. pH-Wert auf 6 bis 7 einstellen. Phase B zugeben und mischen.

Das Beispiel lässt sich mit den Polymeren aus den Beispielen 2, 5, 6 und 7 der EP-A 045 194 wiederholen. Es wird jeweils ein Shampoo mit guten Eigenschaften erhalten.

### Beispiel 70

Verwendung als Filmbildner in einem Desinfektionsspray

Polymer Bsp. 1 EP-A 045 194 werden in 375 demineralisiertem Wasser gelöst und mit 375 g Ethanol versetzt. Anschließend werden in dieser Polymerlösung unter Rühren 100 g Polyvinylpyrrolidon-Jod (PVP-Jod 30/06, BASF)) gelöst und die Zubereitung in Pumpsprayflaschen abgefüllt. Das Desinfektionsspray zeigt sehr gute Filmeigenschaften auf der Haut und weist keinen Jodverlust nach Lagerung unter Stressbedingungen (7 Tage bei 52°C) auf.

Das Beispiel lässt sich mit den Polymeren aus den Beispielen 2, 5, 6 und 7 der EP-A 045 194 wiederholen. Es wird jeweils ein Desinfektionsspray mit guten Eigenschaften erhalten.

## Patentansprüche

1. Kosmetische Mittel enthaltend sulfonierte, Sulfongruppen enthaltende Polymere und in der Kosmetik übliche Zusatzstoffe.

2. Kosmetische Mittel nach Anspruch 1, wobei die sulfonierten, Sulfongruppen enthaltenden Polymere ausgewählt sind aus der Gruppe bestehend aus Polysulfonen, Polyethersulfonen und Polyphenylsulfonen.

3. Kosmetische Mittel nach einem der Ansprüche 1 oder 2, enthaltend die sulfonierten, Sulfongruppen enthaltenden Polymere in einer Menge im Bereich von 0,001 bis 20 Gew.-%, bezogen auf die Gesamtmenge.

4. Kosmetische Mittel nach einem der Ansprüche 1 bis 3, wobei der Sulfonierungsgrad der sulfonierten, Sulfongruppen enthaltenden Polymere größer als 50% ist.

5. Kosmetische Mittel nach einem der Ansprüche 1 bis 4, wobei der Sulfonierungsgrad größer als 80% ist.

6. Kosmetische Mittel nach einem der Ansprüche 1 bis 5, wobei die sulfonierten, Sulfongruppen enthaltenden Polymere teilweise oder vollständig neutralisiert sind.

7. Kosmetische Mittel nach einem der Ansprüche 1 bis 6, wobei die sulfonierten, Sulfongruppen enthaltenden Polymere teilweise oder vollständig als Ammonium- und/oder Alkalimetallsalze vorliegen.

8. Kosmetisches Mittel nach einem der Ansprüche 1 bis 7, wobei die sulfonierten, Sulfongruppen enthaltenden Polymere in einem kosmetisch akzeptablen Lösungsmittel vorliegen und wobei das Lösungsmittel ausgewählt ist aus der Gruppe bestehend aus Wasser, Ethanol, n-Propanol, Isoropanol, n-Butanol, Butan-2-ol, 2-Methylpropan-1-ol, 2-Methylpropan-2-ol, Propylenglykol, Glycerin, Sorbit sowie deren Mischungen.

9. Kosmetisches Mittel nach Anspruch 8 wobei das Lösungsmittel ausgewählt ist aus der Gruppe bestehend aus Wasser, Ethanol, Isopropanol oder Mischungen daraus.

10. Kosmetisches Mittel nach einem der Ansprüche 1 bis 9, wobei das Mittel als Gel, Milch, Creme, Lotion oder Schaum vorliegt.

11. Kosmetisches Mittel nach einem der Ansprüche 1 bis 10, **dadurch gekennzeichnet, daß** es sich um ein Produkt zum Frisieren handelt.

12. Kosmetisches Mittel nach einem der Ansprüche 1 bis 11, wobei sich um bei dem Mittel um ein Produkt für das Haar handelt, das unter den Haarwaschmitteln und Produkten für das Haar ausgewählt ist, die ausgespült oder nicht ausgespült werden und vor oder nach einer Haarwäsche, Färbung, Entfärbung, Dauerwelle oder Entkräuselung aufgetragen werden.

13. Kosmetisches Mittel nach einem der Ansprüche 1 bis 12, wobei das kosmetische Mittel in einem Zerstäuber, Pumpflakon oder Aerosolbehälter konfektioniert ist, um ein Spray, einen Lack oder einen Schaum zu erhalten.

14. Verfahren zur Behandlung von Haaren, **dadurch gekennzeichnet, daß** die Haare mit dem kosmetischen Mittel gemäß einem der Ansprüche 1 bis 13 in Kontakt gebracht werden und gegebenenfalls mit Wasser gespült wird.

15. Verwendung von sulfonierten, Sulfongruppen enthaltenden Polymeren in kosmetischen Zubereitungen.

16. Verwendung von sulfonierten, Sulfongruppen enthaltenden Polymeren in pharmazeutischen Zubereitungen.

17. Verwendung nach Anspruch 16 als Überzugsmittel, Bindemittel, Filmbildner und auch als Matrix für die Wirkstofffreisetzung in pharmazeutischen Zubereitungen, wobei die pharmazeutischen Zubereitungen ausgewählt sind aus der Gruppe bestehend aus Filmtabletten, Filmmikrotabletten, Dragees, überzogenen Pastillen, Kapseln, Kristallen, Kernen, Granulaten oder Pellets.

18. Verwendung nach Anspruch 16 als Filmbildner in Lösungen und Sprays, die, auf der Haut oder der nicht oralen Schleimhaut aufgebracht, einen Film ausbilden.
